# EUROPEAN PATENT APPLICATION

(11) **EP 4 239 649 A1**
(43) Date of publication of application: **06.09.2023**
(21) Application number: 23159859.0
(22) Date of filing: 03.03.2023
(51) Int. Cl.: G16H 50/30, G16H 80/00, G16H 15/00, G16H 40/60

(54) **CARE DELIVERY SYSTEM**

(30) Priority: 04.03.2022 US 202263268864 P
(71) Applicant: Hill-Rom Services, Inc., Batesville, IN 47006-9167 (US)
(72) Inventor: MEYERSON, Craig M., Batesville, 47006-9167 (US); ETCHISON, Patrice, Batesville, 47006-9167 (US); KLEMBCZYK, Jeff, Batesville, 47006-9167 (US); RIBBLE, David Lance, Batesville, 47006-9167 (US); SHIRLEY, Daniel, Batesville, 47006-9167 (US); SUAREZ, Carlos, Batesville, 47006-9167 (US); WOLFE, Glen J., Batesville, 47006-9167 (US)
(74) Representative: Findlay, Alice Rosemary

(57) **Abstract**

A care delivery system detects an event from one or more sensors. The event indicates a safety risk to a patient in a first location. The system determines a clinical workflow based on the safety risk, and one or more rules defined by a healthcare facility in a second location remote from the first location. The system initiates a telehealth consultation based on the clinical workflow. The telehealth consultation includes the patient and a first caregiver in the second location. The system generates an alert identifying the safety risk, and routes the alert to a second caregiver based on the clinical workflow.

## Description

Recent events are prompting hospitals and health care systems to reconsider how and where they deliver care to patients. Some see the patient's home as the safest and most effective option for providing care to certain patients. The hospital-at-home model has become an emerging care option which has been accelerated by recent events.

Hospital-at-home is a care delivery model that enables some patients who need acute-level care to receive it in their homes, rather than in a hospital. This care delivery model has been shown to reduce costs, improve outcomes, and enhance the patient experience.

Providing patient care in a hospital-at-home environment can create needs similar to those in a hospital setting. However, the environment of a patient's home can prevent conventional hospital systems and equipment from being deployed therein.

The present disclosure generally relates to a care delivery system. In one possible configuration, the care delivery system routes alerts to caregivers in accordance with a clinical workflow determined based on a safety risk detected for a patient and one or more rules defined by a healthcare facility remotely located with respect to a location of the patient. Various aspects are described in this disclosure, which include, but are not limited to, the following aspects.

One aspect relates to a care delivery system comprising: one or more sensors monitoring a patient in a first location; at least one processing device in communication with the one or more sensors; and at least one computer readable data storage device storing software instructions that, when executed by the at least one processing device, cause the care delivery system to: detect an event from the one or more sensors, the event indicating a safety risk to the patient in the first location; determine a clinical workflow based on the safety risk and one or more rules defined by a healthcare facility in a second location remote from the first location; initiate a telehealth consultation based on the clinical workflow, the telehealth consultation including the patient and a first caregiver in the second location; generate an alert identifying the safety risk; and route the alert to a second caregiver based on the clinical workflow.

Another aspect relates to a method for providing remote patient care, the method comprising: detecting an event indicating a safety risk to a patient in a first location; determining a clinical workflow based on the safety risk and one or more rules defined by a healthcare facility in a second location remote from the first location; initiating a telehealth consultation in accordance with the clinical workflow, the telehealth consultation including the patient and a first caregiver in the second location; generating an alert identifying the safety risk; and routing the alert to a second caregiver in accordance with the clinical workflow.

Another aspect relates to a non-transitory computer readable storage media including computer readable instructions which, when read and executed by a computing device, cause the computing device to: detect an event indicating a safety risk to a patient in a first location; determine a clinical workflow based on the safety risk and one or more rules defined by a healthcare facility in a second location remote from the first location; initiate a telehealth consultation in accordance with the clinical workflow, the telehealth consultation including the patient and a first caregiver in the second location; generate an alert identifying the safety risk; and route the alert to a second caregiver in accordance with the clinical workflow.

Another aspect relates to a method of providing remote patient care, the method comprising: evaluating a remote environment; determining based on the evaluation of the remote environment whether the remote environment is equipped for the remote patient care; and when the remote environment is not equipped for the remote patient care, delivering a temporary care setting for installation in the remote environment.

The invention will now be further described by way of example with reference to the accompanying drawings, in which:
FIG. 1 schematically illustrates an example of a care delivery system.
FIG. 2 schematically illustrates an example of a method of generating an alert from an event detected by the care delivery system of FIG. 1.
FIG. 3 schematically illustrates an example of an alert that can be generated in accordance with the method of FIG. 2.
FIG. 4 schematically illustrates an example of a method of determining a clinical workflow based on an alert generated by the method of FIG. 2.
FIG. 5 is an isometric view of another example of the care delivery system of FIG. 1.
FIG. 6 is another isometric view of the care delivery system of FIG. 5.
FIG. 7 schematically illustrates an example of the care delivery system of FIG. 5.
FIG. 8 is an interior view of the care delivery system of FIG. 5.
FIG. 9 is an isometric view of another example of the care delivery system of FIG. 1.
FIG. 10 is a view of another example of the care delivery system of FIG. 1.
FIG. 11 illustrates an example of medical equipment that can be deployed inside any of the care delivery systems of FIGS. 1-10.
FIG. 12 schematically illustrates an example of a method of installing a care delivery system in accordance with any of the examples of FIGS. 1-10.
FIG. 13 illustrates an example of a drone being used to survey a property for installing the care delivery system in an example of the method of FIG. 12.
FIG. 14 illustrates an example of a container being loaded by a forklift into an interior of a shipping container of a delivery truck in an example of the method of FIG. 12.
FIG. 15 illustrates another example of a container being loaded by a forklift onto a platform of a delivery truck in an example of the method of FIG. 12.
FIG. 16 is a floorplan view of an example of a temporary care setting including another example of the care delivery system.
FIG. 17 illustrates an example of an interface generated by the care delivery system of FIG. 16 on a computing device to assist removal of medical devices and the medical equipment from the temporary care setting.
FIG. 18 schematically illustrates an example of an anchor used by the care delivery system of FIG. 16.
FIG. 19 schematically illustrates an example of a method of tracking an object inside the temporary care setting of FIG. 16.
FIG. 20 illustrates an example of a permanent acute care setting.
FIG. 21 schematically illustrates an example of a method of providing location based automation for initiating a clinical workflow in the temporary care setting of FIG. 16 and the permanent acute care setting of FIG. 20.
FIG. 22 illustrates an example of a badge worn by a caregiver who is being routed to a patient in accordance with an example of the method of FIG. 21.
FIG. 23 illustrates an example of a video telehealth consultation on a display device located in a room of the patient initiated between the patient and a caregiver in accordance with an example of the method of FIG. 21.
FIG. 24 schematically illustrates an example of a computing device which can be used to implement aspects of the care delivery systems of FIGS. 1-23.

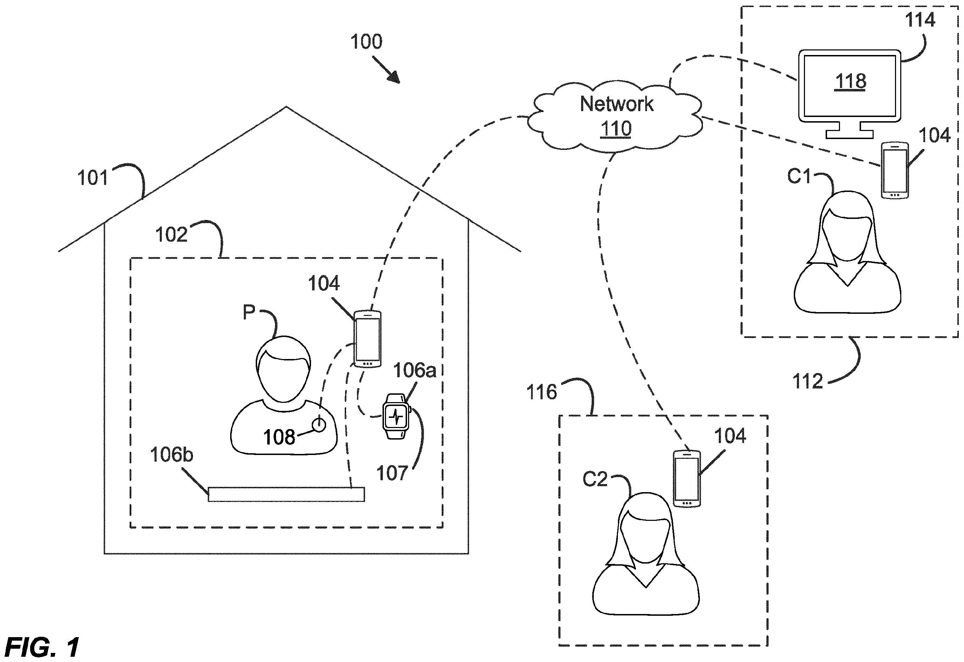

FIG. 1 schematically illustrates an example of a care delivery system 100. As will be described in more detail, the care delivery system 100 brings one or more aspects of a healthcare facility such as a hospital to the patient's home to treat the patient while at home.

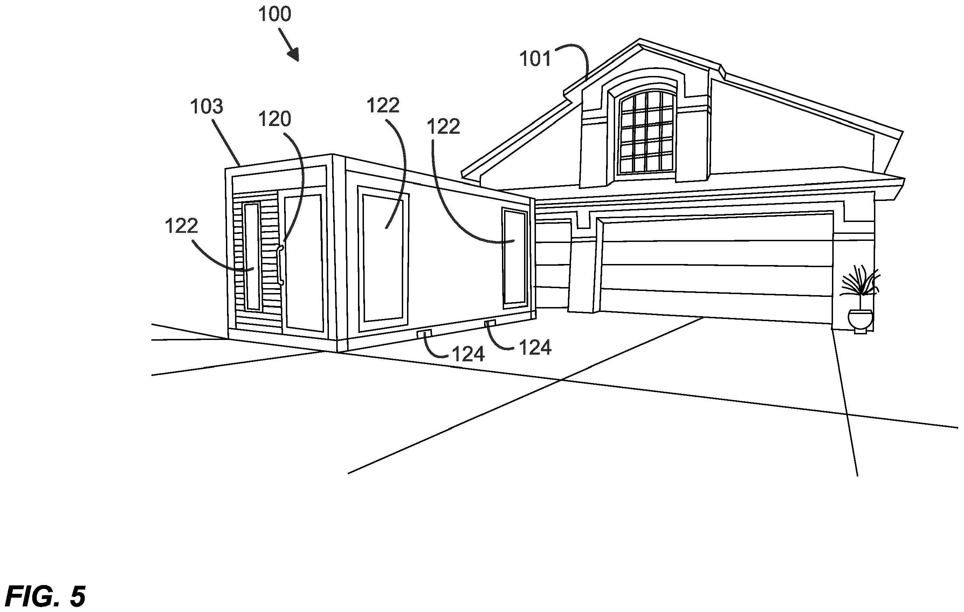

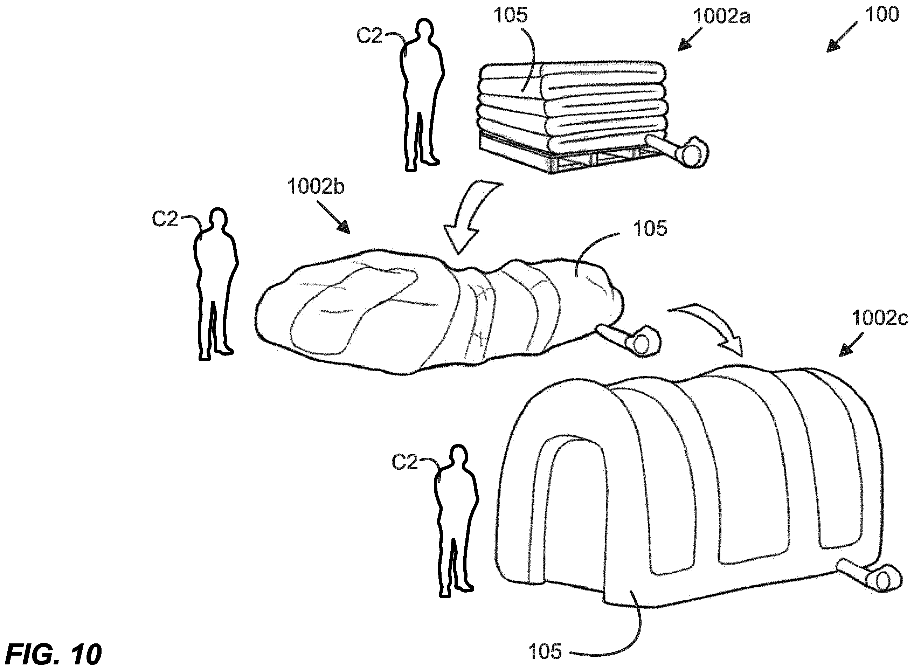

As shown in FIG. 1, a patient P is located in a first location 102. In some examples, the first location 102 is the patient P's home 101. In further examples, the first location 102 is a container 103 delivered for installation outside of the patient P's home that serves as a temporary care setting (see examples shown in FIGS. 5-9). In further examples, the first location 102 is a clean room 105 deployed inside the patient P's home (see example shown in FIG. 10).

A first caregiver C1 is located in a second location 112. The second location 112 is remotely located with respect to the first location 102. Examples of the second location 112 can include healthcare facility such as a hospital that includes a critical care team assigned to the patient P. The first caregiver C1 is a member of the critical care team assigned to the patient P.

In some examples, the critical care team located in the second location 112 is responsible for providing a hospital-at-home medical services to the patient P located in the first location 102. The critical care team can include a group of caregivers including doctors, nurses, and physician assistants, and medical specialists such as, without limitation, pulmonologists, neurologists, cardiologists, oncologists, psychologists, and the like.

A second caregiver C2 is located in a third location 116 such as between the first and second locations 102, 112. In one example, the first caregiver C1 is an inpatient medical professional such as a nurse stationed at a healthcare facility in the second location 112, and the second caregiver C2 is an outpatient or ambulatory medical professional such as an outpatient or ambulatory nurse who visits and treats patients in their homes.

In some examples, the second caregiver C2 is also member of the critical care team. In other examples, the second caregiver C2 is not a member of the critical care team. For example, the second caregiver C2 can include an emergency medical services (EMS) paramedic, or other third party that can be contacted by the first caregiver C1 to provide medical assistance. In further examples, the second caregiver C2 is not a trained medical professional. For example, the second caregiver C2 can be a family member or friend of the patient P.

In the example shown in FIG. 1, the patient P operates a computing device 104 that is connected to one or more sensors inside the first location 102. Also, the first and second caregivers C1, C2 can each carry a computing device 104. Examples of the computing devices 104 can include smartphones, tablet computers, laptop computers, desktop computers, servers (including cloud based servers), and similar computing devices. The computing devices 104 can communicate with each other via a communications network 110.

The computing device 104 of the patient P is connected to one or more sensors including event recorders 106a, 106b and a camera 108. The event recorders 106a, 106b and the camera 108 sync with the computing device 104. For example, the event recorders 106a, 106b and the camera 108 sync with the computing device 104 wirelessly such as through Bluetooth.

In FIG. 1, the computing devices 104 of the patient P and the second caregiver C2 each communicate with an alert system 114 via a communications network 110. The alert system 114 is another example of a computing device such as a cloud based sever. Examples of the communications network 110 can include any type of wired or wireless connections and any combinations thereof, including the Internet, and cellular network connections such as 4G or 5G.

The one or more event recorders 106a, 106b can include a wearable device that records physiological and environmental events of the patient P. An example of a physiological event can include a physiological variable such as heart rate, electrocardiogram (ECG), blood oxygen saturation (SpO2), respiration rate, temperature, and the like falling outside of an upper or lower limit set for the patient P. In some examples, the upper or lower limits are set by the critical care team located in the second location 112. In further examples, the upper and lower limits are personalized for the patient P such as based on the patient P's condition and health history. An example of an environmental event can include the patient P exiting a bed where the patient P is resting, or falling or otherwise having an accident inside the first location 102.

In the example shown in FIG. 1, the event recorder 106a includes a fitness tracker such as a smartwatch that can be worn on the patient P's wrist. In further examples, the event recorder 106 can include sensors such as patches that attach elsewhere to the patient P's body. In further examples, the event recorder 106a includes a pad under the mattress of the bed where the patient P is resting that can record signals indicative of heart rate, respiratory rate, and motion.

The event recorder 106a can monitor health data of the patient P including patient motion and physiological variables including, for example, heart rate, electrocardiogram (ECG), blood oxygen saturation (SpO2), respiration rate, temperature, and the like.

The event recorder 106a can be manually operated to record a physiological or environmental event of the patient P. In such examples, the event recorder 106a includes an input 107 that the patient P can select to record occurrence of an event. The input 107 can include a physical button on the event recorder 106a, a selectable icon on a touchscreen of the event recorder 106a, or can include a voice activated input on the event recorder 106a.

As an illustrative example, the event can include a patient fall. When the input 107 on the event recorder 106a is selected by the patient P, the event is recorded by the event recorder 106a and then pushed by the event recorder 106a to the computing device 104. The computing device 104 can then transmit the event to the alert system 114 via communications network 110.

In addition, or as an alternative to manually selecting the input 107 on the event recorder 106a to record occurrence of an event, the event recorder 106a can automatically record physiological or environmental events pertaining to the patient P without requiring an input from the patient P. For example, the event recorder 106a can automatically detect an event such as when the patient P has fallen. In such examples, the event recorder 106a can automatically record and then push the event to the computing device 104, and the computing device 104 can then transmit the event to the alert system 114 via the communications network 110.

As further shown in FIG. 1, the one or more event recorders can include an event recorder 106b that is a pad positioned on the floor next to the bed where the patient P is resting. The event recorder 106b can detect an event such as when the patient P exits the bed such as to move about the first location 102. The event recorder 106b can automatically record the event, and then push the event to the computing device 104, and the computing device 104 can then transmit the event to the alert system 114 via the communications network 110.

In further examples, the one or more event recorders can include a sensor that detects movements by the patient P inside the first location such as when the patient P exists the bed, leaves the bedroom, enters the bathroom, and the like. In some examples, the sensor is a tag that is attached or worn by the patient P, and fixed reference points positioned throughout the first location 102 detect movements of the patient P via signals received from the tag.

In further examples, the one or more event recorders can include a Global Positioning System (GPS) device that can be used to track the movements of the patient P inside the first location 102. The GPS device can include the computing device 104 or the event recorder 106a.

As shown in FIG. 1, the camera 108 is worn by the patient P. The camera 108 is designed to be small, lightweight, comfortable for wearing, and unobstructive such that it can be worn by the patient P over an extended duration such as during remote patient care and/or hospital-at-home treatment. In some examples, the camera can be worn for about 5 days.

The camera 108 is in communication with the one or more event recorders 106a, 106b. In some examples, the camera 108 indirectly communicates with the one or more event recorders 106a, 106b via the computing device 104. For example, when an event is detected by the one or more event recorders 106a, 106b, or is triggered by the patient P selecting the input 107, the event is transmitted from the one or more event recorders 106a, 106b to the computing device 104, and the computing device 104 then communicates to the camera 108 that an event has been detected or triggered by the one or more event recorders 106a, 106b.

In alternative examples, the camera 108 can communicate directly with the one or more event recorders 106a, 106b. For example, when an event is detected by the one or more event recorders 106a, 106b, or is triggered by the patient P selecting the input 107 on the event recorder 106a, the event can be transmitted directly from the one or more event recorders 106a, 106b to the camera 108. In such examples, the one or more event recorders 106a, 106b and the camera 108 can sync with each other wirelessly such as through a Bluetooth connection.

In one example, the camera 108 continuously records a video stream over an extended duration such as during remote patient care or hospital-at-home treatment. As an example, the camera 108 can continuously record a video stream over a period of five days.

When an event is detected by the one or more event recorders 106a, 106b, an alert is generated. The alert can be generated by an application installed on the computing device 104 of the patient P that communicates with the one or more event recorders 106a, 106b and the camera 108 to generate the alert. The computing device 104 can send the alert via the communications network 110 to the alert system 114 for viewing by the first caregiver C1 in a healthcare facility in the second location 112. In other examples, the alert can be generated by alert system 114 based on data received via the communications network 110 from the computing device 104.

The alert system 114 can generate a clinical workflow for responding to the event. In some examples, the clinical workflow follows a hospital-at-home treatment plan set by the critical care team assigned to the patient P. For example, the clinical workflow is determined based on a safety risk identified from the detected event and one or more rules defined by a healthcare facility in the second location 112, which is remote from the first location 102.

The alert can include the event detected by the one or more event recorders 106a, 106b such as readings of a physiological variable outside of an upper or lower limit set for the patient P, a detection of the patient P exiting their bed, or the patient P falling to the ground. The alert can additionally include a window of a predetermined amount of time from the video stream continuously recorded by the camera 108. For example, the alert can include the video stream recorded during detection of the event, a portion of the video stream recorded before the event is detected, and a portion of the video stream recorded after the event is detected. For example, the alert can include about 30 seconds to about 1 minute of the video stream recorded before the event is detected, the video stream recorded during detection of the event, and the alert can include about 30 seconds to about 1 minute of the video stream recorded after the event is detected. The length of the window, including the portions before and after detection of the event may vary. The alert includes video information captured before, during, and after an event is detected to provide context for the first caregiver C1. Also, the alert can include environmental and physiological data captured during the window (e.g., during the same timeframe as the video information such as about 30 seconds to about 1 minute before the event, during detection of the event, and about 30 seconds to about 1 minute after the event). The context provided in the alert can be used to generate the clinical workflow in response to the detected event.

The video stream recorded by the camera 108 can be stored locally on a memory device of the camera 108, can be stored on a memory device of the computing device 104, or can be stored on a memory device of the alert system 114. In some examples, only a buffer of the video stream is temporarily stored. In such examples, the buffer is used for generating the window of the video stream that includes portions of the video stream recorded before and after the event is detected. The remaining portions of the video stream can be deleted since they are not relevant to the detected event. This can maintain the privacy of the patient P since only the relevant portions of the video stream are sent in the alert for viewing by the first caregiver C1 on the alert system 114. Also, this can reduce memory storage requirements for the memory storage devices of the computing device 104 of the patient P, the camera 108, or the alert system 114.

In some examples, the video processing is performed on the video stream that is continuously recorded by the camera 108 to detect occurrence of an event. The video processing can be performed by the camera 108, or can be performed by the computing device 104 or by the alert system 114. As an illustrative example, video processing can be automatically performed to detect one or more environmental events such as when the patient P exits their bed, when the patient P falls to the ground, or when the patient P leaves the first location 102.

In alternative examples, the camera 108 does not continuously record a video stream. Instead, the camera 108 is turned off or otherwise remains inactive until an event is detected by the one or more event recorders 106a, 106b. When an event is detected, a signal is sent either from the computing device 104, or directly from the one or more event recorders 106a, 106b, to activate the camera 108 to start recording the video stream. Thereafter, the video stream can be transmitted along with the alert to the alert system 114 for viewing by the first caregiver C1.

The alert can be classified into one or more priority levels based on the detected event, and the condition and health history of the patient P. The classification of the alert can be used to generate a clinical workflow in accordance with a hospital-at-home treatment plan.

As an illustrative example, when the event is the patient P exiting the bed following a hip replacement surgery, the clinical workflow can include a phone call or video call from the first caregiver C1 to the patient P requesting that the patient P return to their bed because leaving their bed unassisted can be unsafe. If the patient P ignores the phone call or otherwise does not return to their bed as requested by the first caregiver C1, the alert can be escalated such that the clinical workflow can include calling a designated family member of the patient P to request assistance in returning the patient P to their bed. As another illustrative example, when event is the patient P falling to the ground, the clinical workflow can include a phone call to the second caregiver C2 such as an EMS paramedic who can provided emergency medical services. Additional examples of clinical workflows that can be performed are contemplated.

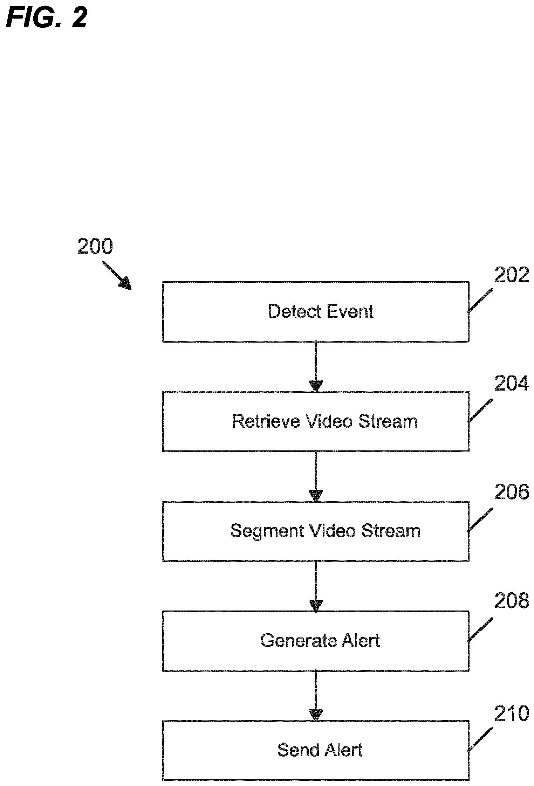

FIG. 2 schematically illustrates an example of a method 200 of generating an alert from an event detected by the care delivery system 100. The first location 102 can include the patient P's home 101, a container 103 delivered outside of the patient P's home (see FIGS. 5-9), or a clean room 105 deployed inside the patient P's home (see FIG. 10). The method 200 can be performed by an application installed on the computing device 104 of the patient P that is in communication with the one or more event recorders 106a, 106b and the camera 108. In alternative examples, the method 200 can be performed by the alert system 114 based on data received over the communications network 110 from the computing device 104 of the patient P.

The method 200 includes an operation 202 of detecting an event inside the first location 102. In some examples, the event is a safety risk such as a physiological or environmental event detected by the one or more event recorders 106a, 106b. In some examples, the event is detected by video processing the video stream recorded by the camera 108.

Next, the method 200 includes an operation 204 of retrieving the video stream recorded by the camera 108 following detection of the event. As described above, the video stream can be retrieved by the computing device 104 of the patient P from the camera 108. Also, the video stream can be retrieved by the alert system 114 from data received from the computing device 104 of the patient P over the communications network 110.

Next, the method 200 includes an operation 206 of segmenting a window of the video stream recorded by the camera 108. Operation 206 includes segmenting the window to include a portion of the video stream recorded before the event is detected and a portion of the video stream recorded after the event is detected. The window can include about 30 seconds to about 1 minute of the video stream recorded before the event is detected, and the alert can include about 30 seconds to about 1 minute of the video stream recorded after the event is detected. The length of the window and the portions before and after the event is detected may vary.

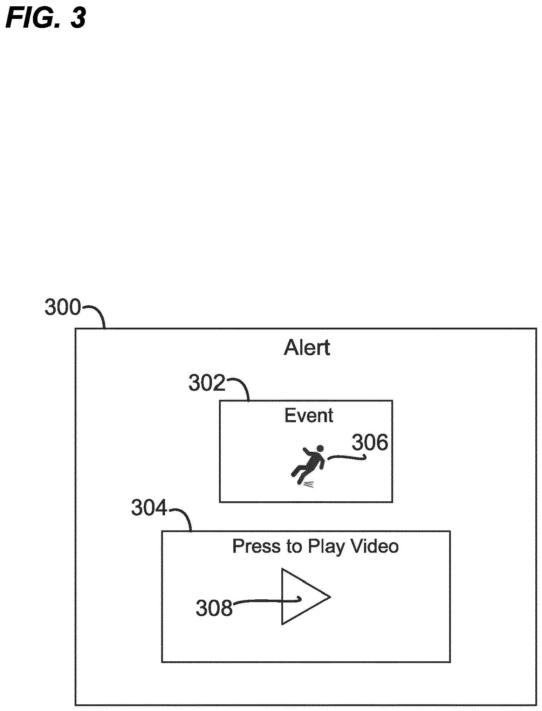

Next, the method 200 includes an operation 208 of generating an alert. FIG. 3 schematically illustrates an example of an alert 300 that can be generated in accordance with the method 200. The alert 300 can identify an event 302 detected in operation 202 and a window of the video stream 304 segmented in operation 206. The event 302 can include a textual or visual descriptor 306 of the event such as a patient fall. The window of the video stream 304 can include an icon 308 to play the window of the video stream 304 which can include portions of the video stream captured before and after the event 302 is detected. Additionally, the alert can also include environmental and physiological data captured during the window (e.g., during the same timeframe as the video stream 304). Such information can be displayed inside the event 302. The window of the video stream 304 (as well as the environmental and physiological data) can provide context for the event 302 displayed in the alert 300 for viewing by the first and second caregivers C1, C2, and can be used to generate a clinical workflow.

In some examples, the method 200 includes an operation 210 of sending the alert 300 to another device. In examples where the method 200 is performed on the computing device 104 of the patent P, operation 210 can include sending the alert 300 from the computing device 104 of the patient P to the alert system 114 via the communications network 110. In examples where the method 200 is performed on the alert system 114, operation 210 can include sending the alert 300 for display on a display device 118 in the alert system 114 for viewing by the first caregiver C1 in the second location 112, and/or sending the alert 300 from the alert system 114 to one or more computing devices 104 operated by the first and second caregivers C1, C2.

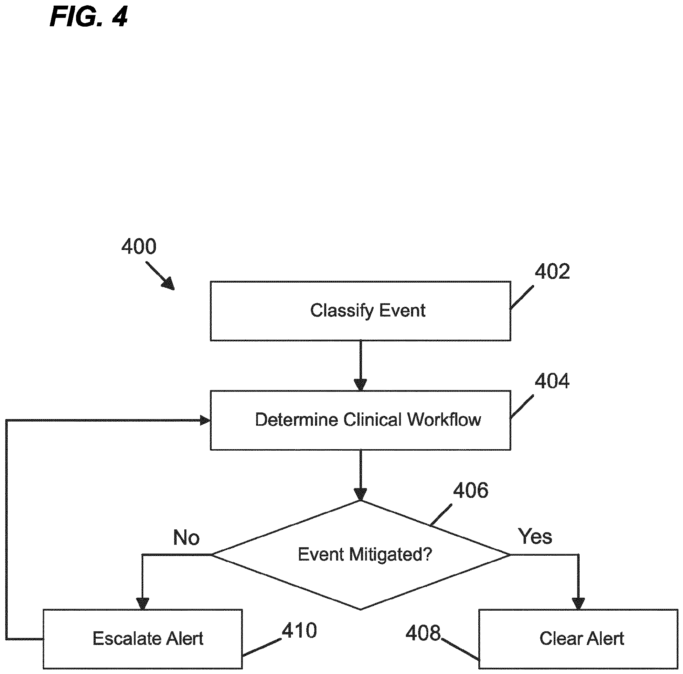

FIG. 4 schematically illustrates an example of a method 400 of determining a clinical workflow based on an event detected inside the first location 102. In some examples, the method 400 is performed by the alert system 114 that is remotely located with respect to the patient P.

The method 400 includes an operation 402 of classifying the detected event into one or more categories. In some examples, the one or more categories are priority levels based on the event and the health condition of the patient P. Examples of the priority levels can include critical, moderate, and noncritical. As an illustrative example, an event is classified as critical when the event is a patient fall, and the patient P has advanced age and/or one or more comorbidities. As another example, the event is classified as moderate when the event is a bed exit, and a care plan requires the patient P to remain resting in bed. As another example, the event is classified as noncritical when the event is a lost signal from disconnection of an event recorder, and the patient P has a stable health condition. Additional examples are contemplated.

Next, the method 400 includes an operation 404 of determining a clinical workflow based on the priority category of the event (e.g., a safety risk identified from the detected event) and one or more rules defined by a healthcare facility in the second location 112.

As an illustrative example, a clinical workflow can include a telehealth consultation from the first caregiver C1 to the patient P for an event classified as noncritical. As another example, a clinical workflow can include a telehealth consultation from the first caregiver C1 to the second caregiver C2 who is a family member of the patient P for an event classified as moderate. As another example, a clinical workflow can include a telehealth consultation from the first caregiver C1 to the second caregiver C2 who is an EMS paramedic for emergency medical assistance when the event is classified as critical. Additional examples are contemplated.

Next, the method 400 includes an operation 406 of determining whether the event is mitigated. As an illustrative example, when the event is the patient P exiting the bed following a hip replacement surgery, the clinical workflow can include a telehealth consultation from the first caregiver C1 to the patient P requesting that the patient P return to their bed because leaving their bed unassisted is unsafe. The telehealth consultation can provide medical services consistent with a hospital-at-home treatment plan set by the critical care team assigned to the patient P. In some examples, the telehealth consultation is a video call.

When the patient returns to their bed following the telehealth call (i.e., "Yes" in operation 406), the method 400 can proceed to an operation 408 of clearing the alert. When the patient P does not answer or does not return to their bed following the telehealth consultation with the first caregiver C1 (i.e., "No" in operation 406), the method 400 can proceed to an operation 410 of escalating the alert 300 such as to increase the priority level from noncritical to moderate, or from moderate to critical. The method 400 then returns to operation 404 to determine a new clinical workflow based on the escalated priority level. As an illustrative example, the new clinical workflow determined in operation 404 can include calling a designated family member of the patient P to request assistance in returning the patient P to their bed.

As a further example, when the family member of the patient P does not answer the telehealth call (i.e., "No" in operation 406), or another event such as a patient fall is detected, the method 400 can proceed again to operation 410 of escalating the alert 300 such as to increase the priority level from moderate to critical. The method 400 then returns to operation 404 to determine a new clinical workflow based on the escalated priority level. As an illustrative example, the new clinical workflow can include calling the second caregiver C2 to go to the first location 102 to check on the patient P or to provide emergency medical services.

The events detected by the one or more event recorders 106a, 106b are also combinable to escalate an alert. As an illustrative example, an alert is generated when an event is recoded that detects the patient P has exited the bed, and the alert is escalated when a subsequent event is recorded that detects the patient P has left the first location 102.

FIG. 5 is an isometric view of another example of the care delivery system 100. In this example, the care delivery system 100 is a hospital-at-home system that includes a container 103 deployed outside of the patient P's home 101. The container 103 is a single patient decentralized care setting that is customizable based on the needs of the patient P. The container 103 can be removed from the patient P's property after treatment is complete, and is reusable. The container 103 can increase the capacity of a healthcare facility such as a hospital when under a surge such as when there are not enough space to accommodate all patients. A surge may occur due to a natural disaster such as a hurricane, or may be due to spready of an infectious disease.

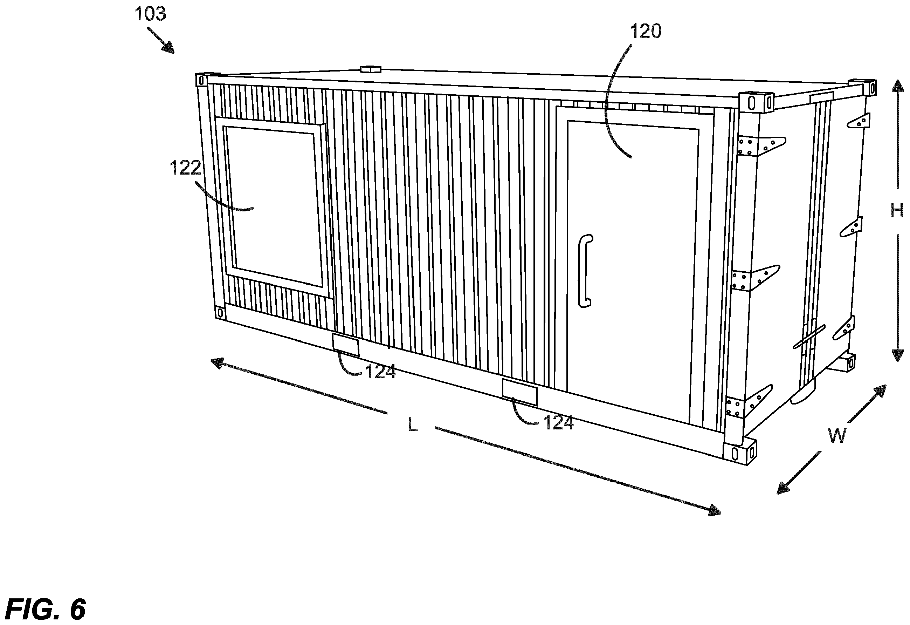

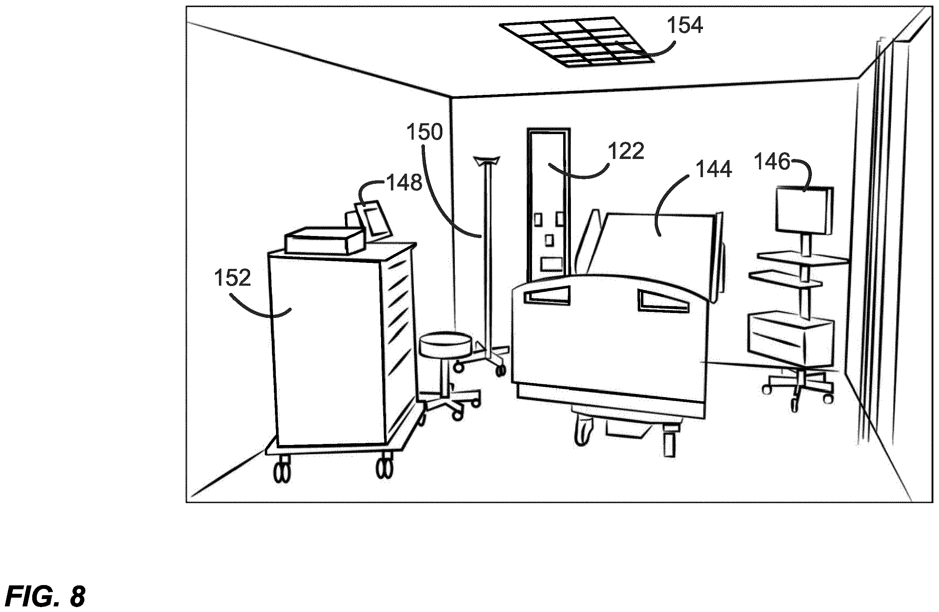

FIG. 6 is an exterior isometric view of another example of the container 103. As shown in FIGS. 5 and 6, the container 103 includes a door 120 to allow the patient P to enter the container for patient care, and to leave the container 103 when the patient care is completed. The container 103 can include one or more windows 122 to allow natural light to enter the container. Also, the container 103 can include apertures 124 to allow forks of a fork lift to slot therein to pick up the container 103 such as for loading the container 103 onto a truck and unloading the container 103 from the truck for deployment in an area proximate the patient P's home 101 (e.g., driveway, front yard, or back yard of the patient P's home 101). In some examples, the container 103 can include wheels (see example shown in FIG. 8) that allow the container 103 to be wheeled onto a property or space proximate the patient P's home 101.

In FIG. 6, the container 103 is a shipping container converted to provide medical care. In this example, the container 103 is a durable closed rectangular box having a standardized size. For example, the container 103 can have a width W of about 8 feet, a length L of about 20 feet, and a height H of about 8 feet. Advantages of the container 103 include that the container 103 supports intermodal freight transport, such that the container 103 can be transported across different modes of transportation (e.g., from ship to rail to truck). The container 103 is made of durable rust-retardant steel, allowing the container 103 to be reused after deployment. The materials and manufacturing costs for the container 103 can be reduced by converting a pre-existing shipping container, instead of building the container 103 from the ground up.

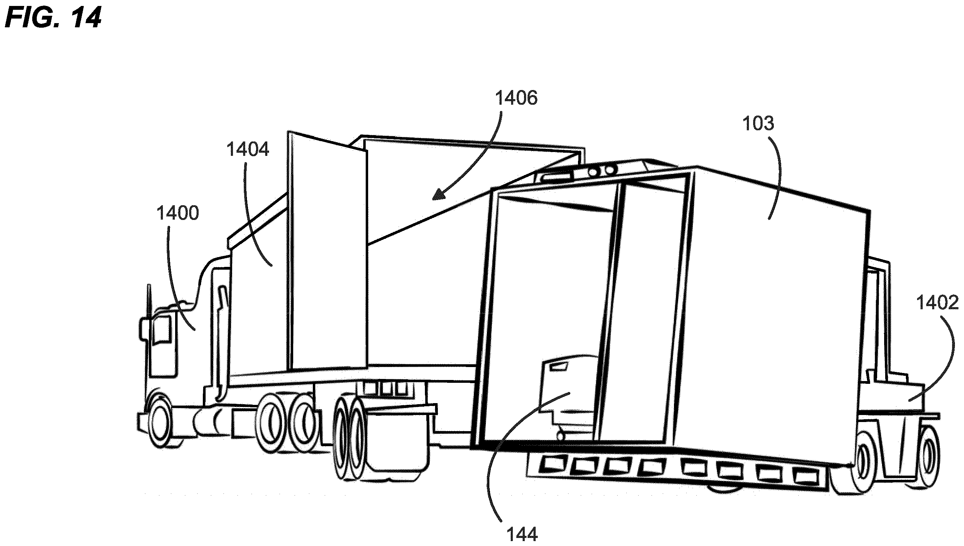

In alternative examples, the container 103 is a trailer unit that is constructed for deployment outside of the patient P's home. The trailer unit can be constructed to fit inside a shipping container for transport. An example of this configuration is shown in FIG. 14.

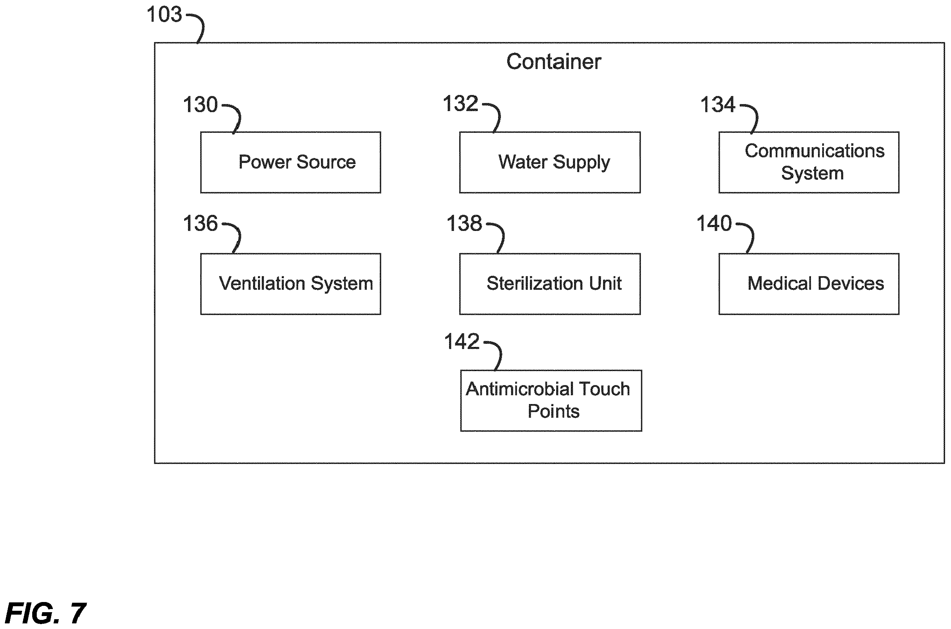

FIG. 7 schematically illustrates example components of the container 103. It is contemplated that in some examples the container 103 can include fewer components than all of the components shown in FIG. 7, or can include additional components not shown in FIG. 7.

The container 103 includes a power source 130 for supplying electrical power to devices inside the container. The container 103 includes one or more electrical outlets for one or more devices including medical devices to plug into to receive electrical power from the power source 130. In some examples, the power source 130 includes a power generator. In further examples, the power source 130 includes a battery for energy storage. In further examples, the power source 130 includes a connector to connect the container 103 to a power grid.

The container 103 can further include a water supply 132 for supplying water to one or more faucets or outlets inside the container. In some examples, the water supply 132 includes a water hookup that can connect to a water supply outside of the container. In some examples, the water supply 132 includes a water filtration system to filter the water from the water supply. In further examples, the water supply 132 can include a water tank.

The container 103 can further include a communications system 134 for connecting one or more devices inside the container to a remote care facility such as a hospital and/or to remote systems such as the alert system 114 shown in FIG. 1. The communications system 134 can include a router that provides internet connection via the communications network 110. The internet connection can facilitate a telehealth consultation with one or more caregivers.

The container 103 can further include a ventilation system 136 that filters the air inside the container 103. In some examples, the ventilation system 136 creates negative air pressure inside the container 103 allowing air to flow into the container but not escape from the container, and thereby prevent contamination of the air outside of the container 103.

The container 103 further includes a sterilization unit 138 that operates to sterilize the interior of the container for re-deployment and use by another patient. In some examples, the sterilization unit 138 includes one or more ultraviolet lights positioned on the interior of the container 103 such as on the ceiling that emit ultraviolet light to sterilize the interior of the container once the patient P has completed their treatment and has left the container 103.

The container 103 further includes medical devices 140 that are selected based on the patient P's condition and care needs. The medical devices 140 can include a patient support system 144 (see FIG. 8) such as a hospital bed. The medical devices 140 can further include a monitor device 146 (see FIG. 8) such as a spot monitor. The patient support system and monitor device can each support one or more sensors for monitoring physiological variables of the patient P such as an electrocardiogram (ECG) sensor, a blood oxygen saturation/pulse oximeter (SpO2) sensor, a blood pressure sensor for measuring systolic and diastolic blood pressure, a heart rate sensor, a respiration rate sensor, an end tidal carbon dioxide (etCO2) sensor, and the like. The medical devices 140 can also include medical intervention devices such as an infusion pump for delivery of fluids including nutrients and medications, a respiratory ventilator, a dialysis machine, and similar types of devices. The medical devices 140 can further include medical gasses such as an oxygen supply tank for when the patient P is connected to the respiratory ventilator for treatment of a respiratory condition.

The medical devices 140 further include telehealth equipment 148 (see FIG. 8) such as a display and microphone/speaker unit to allow the patient P and/or the second caregiver C2 to have a telehealth consultation with the first caregiver C1 and/or other members of the critical care team assigned to the patient P that are remotely located with respect to the container 103.

The container 103 can further include antimicrobial touch points 142 to reduce the spread of bacteria inside the interior of the container. In some examples, the antimicrobial touch points 142 include antimicrobial copper film or similar types of antimicrobial films.

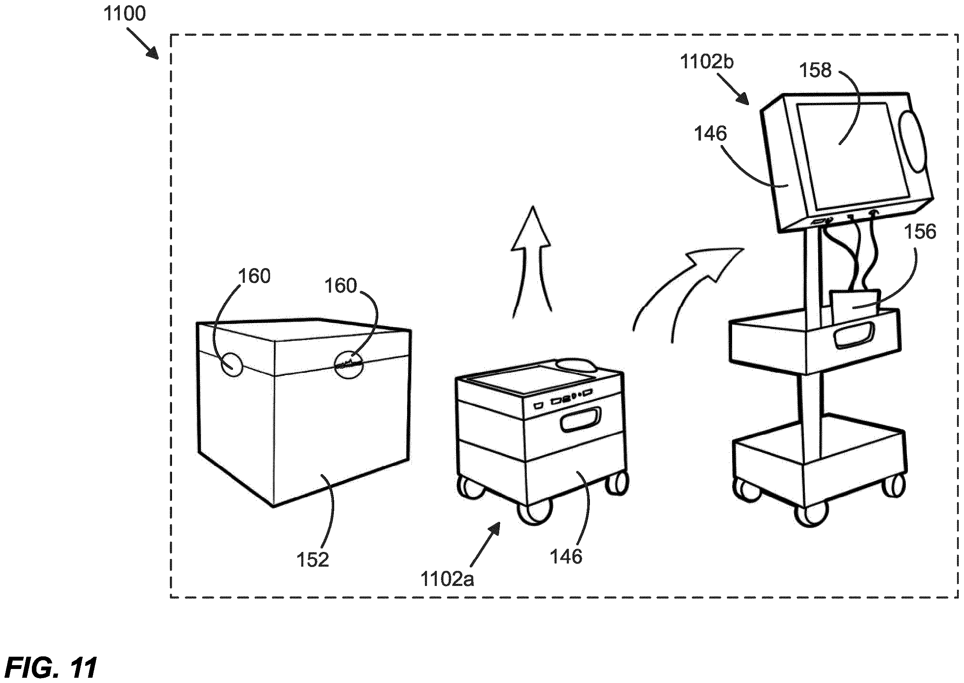

FIG. 8 is an interior view of the container 103. The interior of the container 103 includes the medical devices 140 such as the patient support system 144, the monitor device 146, and the telehealth equipment 148. In the example shown in FIG. 8, the telehealth equipment 148 is separate from the monitor device 146. In other examples, the monitor device 146 and the telehealth equipment 148 can be combined into a single device (e.g., see FIG. 11) to reduce space requirements for the medical devices 140 inside the container 103.

The medical devices 140 can further include a portable stand 150 to support an intravenous (IV) fluid drip. Also, a crate 152 can be included to house medical equipment and supplies that are selected based on the patient P's condition and care needs.

The interior of the container 103 can further include a window 122 to allow natural light to enter into the interior of the container 103, and one or more ceiling light fixtures 154. In some instances, the one or more ceiling light fixtures 154 include the one or more ultraviolet lights that emit ultraviolet light to sterilize the interior of the container 103.

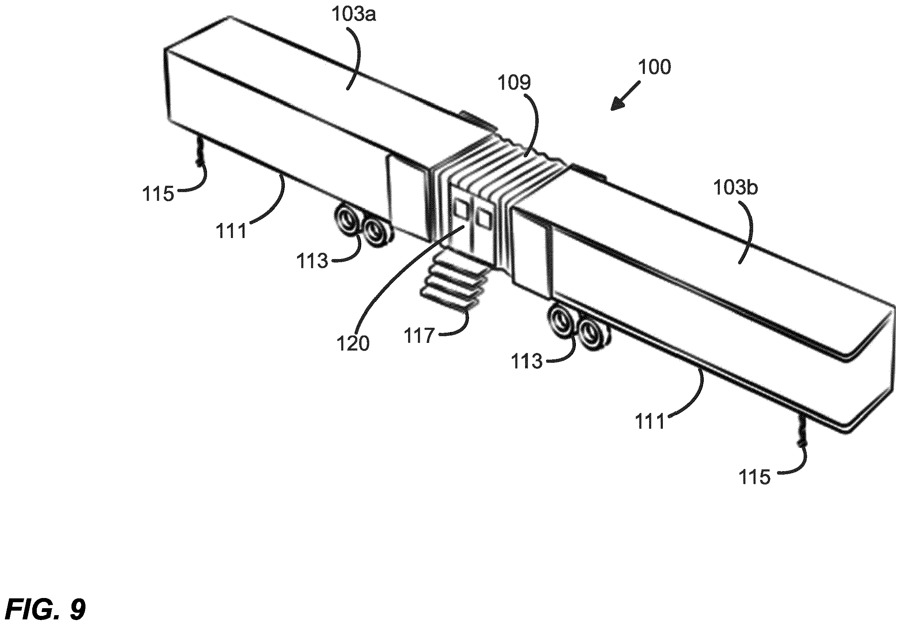

FIG. 9 is an isometric view of another example of the care delivery system 100. In this example, the care delivery system 100 includes two of the containers 103 joined together. This arrangement can increase the interior volume and capacity of the care delivery system.

In the example shown in FIG. 9, an opening of a first container 103a is joined to an opening of a second container 103b by a connector 109. In some examples, the connector 109 provides an open gangway or corridor connection between the first and second containers 103a, 103b. In other examples, the connector 109 partitions the first and second containers 103a, 103b from one another such that the first and second containers 103a, 103b have separate interiors. The connector 109 includes doors 120 such as double leaf doors that provide an entrance into the interiors of the first and second containers 103a, 103b. In one configuration, the first and second containers 103a, 103b can each be occupied by a different patient such that the configuration shown in FIG. 9 can double the capacity of the care delivery system.

As further shown in FIG. 9, the first and second containers 103a, 103b are mounted onto a chassis 111 that includes wheels 113. The chassis 111 can also include retractable stands 115 to support the first and second containers 103a, 130b. Also, retractable steps 117 are included on or connected to the connector 109 to allow persons such as the patient P and the second caregiver C2 to enter the first and second containers 103a, 103b. When the patient care is completed, the connector 109 can be disassembled from the first and second containers 103a, 103b, and each of the first and second containers 103a, 103b can be hauled away from the deployment location by a truck for return to a cleaning facility and redeployment.

FIG. 10 is an isometric view of another example of the care delivery system 100. In this example, the care delivery system 100 includes a clean room 105 that is designed for installation and deployment inside the patient P's home 101. The clean room 105 is designed for temporary use such that it can be assembled and disassembled inside the patient P's home such as by the second caregiver C2. In the example shown in FIG. 10, the clean room 105 is inflatable from a deflated state 1002a, to a partially inflated state 1002b, to a fully inflated state 1002c.

When assembled (e.g., inflated), the clean room 105 provides a controlled and sterilized environment inside the patient P's home 101. When the patient care is completed, the clean room 105 can be disassembled (e.g., deflated) and packaged for removal from the patient P's home. Thereafter, the clean room 105 can be cleaned and sterilized for redeployment. The assembly and disassembly of the clean room 105 can help facilitate storage of the care delivery system 1000 when not in use, and can also help transport the care delivery system 1000.

FIG. 11 illustrates an example of medical equipment 1100 that can be deployed inside the care delivery systems of FIGS. 1-10 including in the patient P's home 101, the containers 103, and the clean room 105. The medical equipment 1100 can allow for remote care of the patient P while inside the patient P's home, 101, the container 103, or the clean room 105. For example, the medical equipment 1100 can be used to perform specialized medical procedures and applications within the patient P's home 101, the containers 103, or the clean room 105. As an illustrative example, the medical equipment 1100 can be used to perform emergency department, operating room, intensive care unit (ICU), isolation, screening and observation, healthy patient discharge quarantine, diagnostic imaging, and primary care procedures and applications inside the patient P's home 101, the containers 103, and/or the clean room 105.

The medical equipment 1100 includes the monitor device 146 that supports one or more sensors 156 for monitoring physiological variables of the patient P such as an electrocardiogram (ECG) sensor, a blood oxygen saturation/pulse oximeter (SpO2) sensor, a blood pressure sensor for measuring systolic and diastolic blood pressure, a heart rate sensor, a respiration rate sensor, an end tidal carbon dioxide (etCO2) sensor, and the like.

The monitor device 146 can further include a telehealth unit 158 to allow the patient P and/or the second caregiver C2 to have a telehealth consultation with the first caregiver C1 and/or with other members of the critical care team assigned to the patient P that are remotely located. The telehealth unit 158 includes at least a display screen and a speaker/microphone unit. In some examples, the telehealth unit 158 further includes a video camera to allow for two-way video conferencing. In some further examples, the telehealth unit 158 can include a wireless hotspot for connection to remote systems such as the alert system 114 shown in FIG. 1. In the example shown in FIG. 8, the monitor device 146 combines the functions of monitoring physiological variables and providing telehealth consultations into a single device. This can reduce space requirements for deploying the care delivery systems of FIGS. 1-10.

In some examples, the telehealth unit 158 is connected to an electronic health record (EHR) system of a remote healthcare facility such as a hospital. In such examples, the telehealth unit 158 can store readings of one or more physiological variables of the patient P that are captured by the one or more sensors 156 into the patient P's electronic health record (EHR) for viewing by one or more remotely located medical specialists. This can eliminate the need for a medical specialist to physically visit the patient P while the patient P is receiving medical treatment inside the patient P's home 101, the container 103, or the clean room 105.

As shown in the example of FIG. 11, the monitor device 146 can be stored and transported while in a collapsed position 1102a. When deployed inside patient P's home 101, the containers 103, and/or the clean room 105, the monitor device 146 can be extended into an opened position 1102b. The collapsed position 1102a can help facilitate storage of the monitor device 146 in a distribution center, and can help transport from the distribution center to a deployment location such as the patient P's home 101, a container 103, or a clean room 105 because the monitor device 146 occupies less space when in the collapsed position 1102a.

As further shown in the example of FIG. 11, the medical equipment 1100 can include the crate 152 that houses medical supplies that are selected based on the patient P's condition and care needs. The crate 152 can house a clinical technology packet that allows a person such as the second caregiver C2 to setup remote care for the patient P. The crate 152 can also include test kits and other diagnostic tools for obtaining samples from the patient P. In some examples, the crate 152 including the test kits are designed for drone pickup and drop-off.

The crate 152 includes a tamper seal 160 that when unbroken indicates that the contents of the crate 152 have not been accessed or contaminated. When the tamper seal 160 is broken, this can indicate that the contents of the crate 152 have been accessed and potentially contaminated such as by the patient P or the second caregiver C2. Thus, the tamper seal 160 is broken, this can indicate a need to sterilize or discard any of the remaining contents of the crate 152 after the treatment of the patient P has been completed.

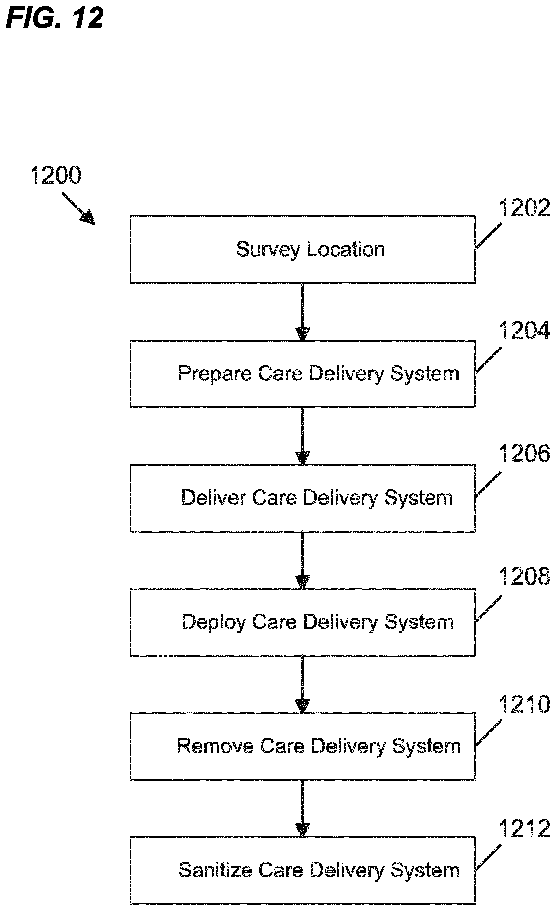

FIG. 12 schematically illustrates an example of a method 1200 of delivering and installing the care delivery systems of FIGS. 1-10. Advantages of the method 1200 can include providing cost-effective surge planning for a healthcare facility such as hospital without requiring capital investment such as building a new department or floor within an existing building to increase surge capacity. Additionally, the method 1200 can result in better care and safety for patients by allowing for treatment inside of or next to a familiar environment such as the patient P's home. The method 1200 can also reduce exposure of the patient P to contagious diseases during a pandemic and other potentially dangerous exposures by allowing the patient P to receive care in a decentralized care setting that does not require the patient P to physically enter the healthcare facility where other patients are being treated. Also, the method 1200 can reduce the exposure of caregivers to contagious diseases during a pandemic by allowing the caregivers (e.g., the first caregiver C1) to provide remote care and telehealth consultations without having to physically be present in the space where the patient P is being treated.

As shown in FIG. 12, the method 1200 includes an operation 1202 of surveying a location for deploying a care delivery system. Operation 1202 can include a site visit by a surveyor or a caregiver to determine the physical constraints of the location such as the interior dimensions of the patient P's home, or the size of the property next to the patient P's home such as the size of the driveway, front yard, and/or back yard.

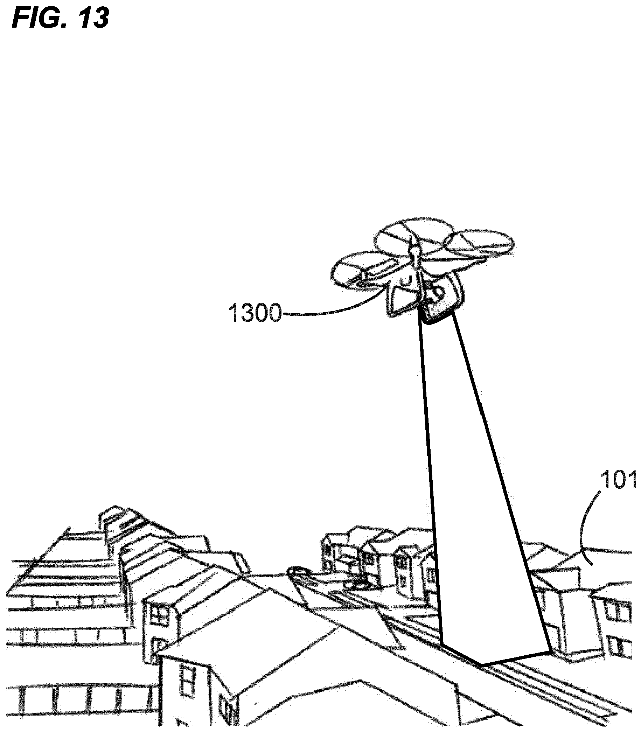

In examples where the care delivery system 100 includes a container 103 for installation outside of the patient P's home, such as in the examples shown in FIGS. 5-9, operation 1202 can include using a drone to survey the property surrounding the patient P's home. FIG. 13 illustrates an example of a drone 1300 being used in operation 1202 to survey a property for installing the care delivery system that includes a container 103. In further examples, the drone 1300 can be used to deliver medical supplies, devices, and or equipment outside of the patient P's home, and to retrieve from outside of the patient P's home medical tests and samples collected from the patient P. In further examples, satellite images can be used to survey a property for installing a container 103 next to the patient P's home.

Next, the method 1200 includes an operation 1204 of preparing the care delivery system based on the survey of the location and on the care needs of the patient P. Operation 1204 can including building a container 103 having dimensions that fit within the property outside of the patient P's home, or preparing a clean room 105 that can fit inside the patient P's home. Operation 1204 can also include preparing one or more of the medical devices 140 and medical equipment 1100 based on the patient P's care needs for delivery.

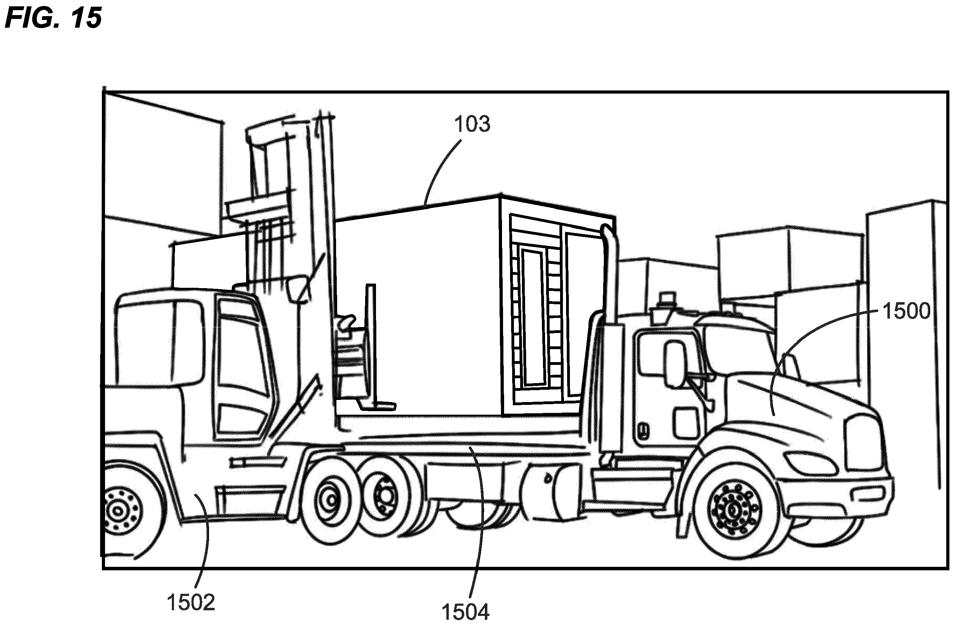

Next, the method 1200 includes an operation 1206 of delivering the care delivery system including the medical devices 140 and medical equipment 1100 to the location. FIG. 14 illustrates an example of a container 103 being loaded by a forklift 1402 into an interior 1406 of a shipping container 1404 of a delivery truck 1400 for transport to the location. FIG. 15 illustrates another example of a container 103 being loaded by a forklift 1502 onto a platform 1504 of a delivery truck 1500. In both of the examples shown in FIGS. 14 and 15, the containers 103 are pre-fabricated for deployment and installation outside of the patient P's home.

Referring back to FIG. 12, the method 1200 further includes an operation 1208 of deploying the care delivery system. In examples where the care delivery system includes a container 103, operation 1208 can include placing the container onto the property next to the patient P's home such as a driveway, a front yard, or a back yard. In some examples, operation 1208 can further include preparing the container 103 for use such as by connecting a water hookup to a water supply. In examples where the care delivery system includes a clean room 105, operation 1208 can include assembling the clean room 105 inside the patient P's home such as by inflating the clean room 105 from the deflated state 1002a to the fully inflated state 1002c. Operation 1208 can further include unloading and preparing one or more of the medical devices 140 and/or the medical equipment 1100 for treatment of the patient P.

Next, the method 1200 includes an operation 1210 of removing the care delivery system once the treatment of the patient P is complete. For example, operation 1210 can include packing the medical devices 140 and/or the medical equipment 1100, and/or disassembling the clean room 105 such as by deflating the clean room 105 from the fully inflated state 1002c to the deflated state 1002a. In examples where the care delivery system includes a container 103, operation 1210 can include disconnecting the water hookup from the water supply and loading the container 103 onto a truck for removal away from the property of the patient P's home.

In some examples, method 1200 can further include an operation 1212 of sanitizing the components of the care delivery system for redeployment. For example, the container 103, clean room 105, medical devices 140, medical equipment 1100, and the like can be cleaned and sanitized for redeployment to another home or property for the treatment of another patient.

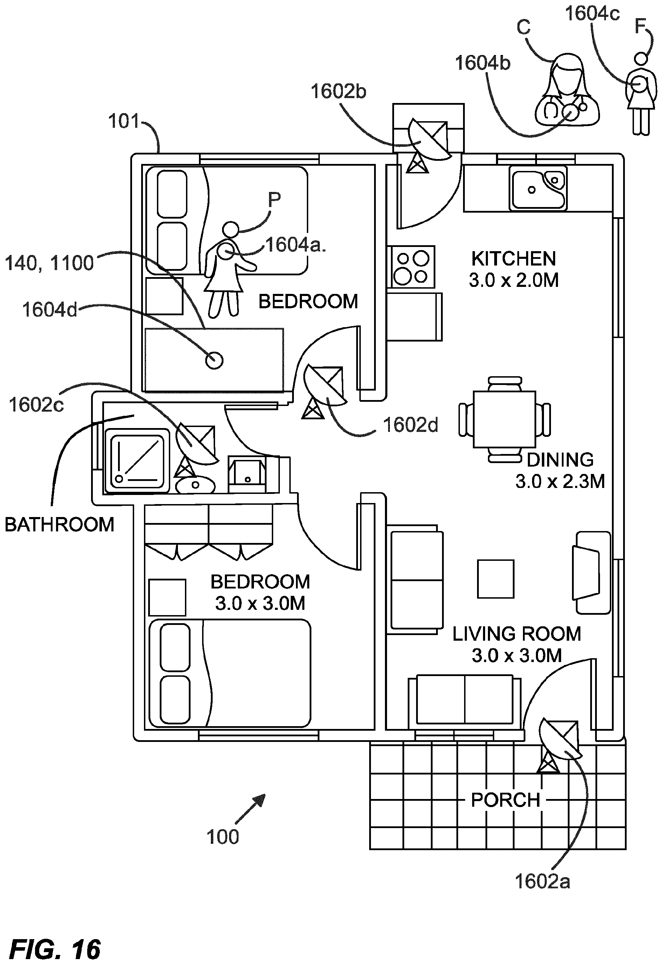

FIG. 16 is floorplan view of a temporary care setting such as the patient P's home 101 in accordance with another example of the care delivery system 100. In this example, the care delivery system 100 includes one or more anchors 1602 that are each a fixed reference point positioned in a strategic location inside the patient P's home 101. For example, an anchor 1602a is positioned at the front entrance of the patient P's home 101, an anchor 1602b is positioned at the rear entrance of the patient P's home 101, an anchor 1602c is positioned in the doorway of the bedroom, and an anchor 1602d is positioned inside the bathroom. As will be described in more detail, the anchors 1602 allow the care delivery system 100 to recognize when tagged caregivers arrive and depart from the patient P's home. As another example, the anchors 1602 allow the care delivery system 100 to recognize and track patient activities such as toileting.

In some examples, a layout of the anchors 1602 positioned in the patient P's home 101 can be tested prior to a start of a hospital-at-home treatment plan. For example, after initial deployment of the anchors 1602, an installer can move through the patient P's home 101 (i.e., the patient environment) in scripted routes based on potential use cases to confirm that the layout is correct or to suggest needed changes (e.g., anchor location, use of additional anchors, etc.).

While FIG. 16 shows the anchors 1602 positioned in various locations inside the patient P's home 101, the anchors 1602 can be used in various environments such as inside a container 103 that is deployed outside of the patient P's home 101 to monitor the location and movement of the patient P inside the container 103, to monitor the presence and location of the caregiver C inside the container 103, to monitor the presence and location of the family member F inside the container 103, and to monitor the location and movement of the medical devices 140 and the medical equipment 1100 that are delivered to the container 103.

In further examples, the anchors 1602 can be used in additional types of care delivery settings outside of a hospital setting. For example, the anchors 1602 can be temporarily positioned inside an assisted living community when a resident of the community has an elevated need for surveillance based on a change in health condition or medication.

The care delivery system 100 further includes tags 1604 attached to one or more objects. The tags 1604 are wireless tags that include an identifier that identifies the object to which the tag is attached to. In some examples, the identifier is generic such that it generically identifies the patient P, a caregiver C, a family member F, and medical devices and equipment. In other examples, the identifier identifies the patient P, the caregiver C, the family member F, and the medical devices and equipment with greater specificity such as by identifying the name of the caregiver C (e.g., "Dr. John Smith"), the role of the family member F (e.g., spouse, daughter, son, sister, brother, etc.), and the type of medical devices and equipment.

The care delivery system 100 uses signal communications between the anchors 1602 and the tags 1604 to track the location and movement of the objects. For example, the signal communications between the anchors 1602 and the tags 1604 is used by the care delivery system 100 to track the location and movement of persons inside the patient P's home 101 such as the patient P, the caregiver C, and the family member F, and to track the location and movement of material objects inside the patient P's home 101 such as the medical devices 140 and the medical equipment 1100 delivered to the patient P's home 101 (e.g., see FIGS. 7, 8, and 11).

The signal communications between the anchors 1602 and the tags 1604 is wireless and can be based on one or more wireless technologies such as radio frequency (RF), infrared (IR), ultrasound, ultra-wideband (UWB), Wi-Fi, Bluetooth, and similar wireless technologies. In some examples, the signal communications between the anchors 1602 and the tags 1604 include the anchors 1602 receiving wireless signals from the tags 1604 such that the anchors 1602 are receivers and the tags 1604 are transmitters. In other examples, the signal communications between the anchors 1602 and the tags 1604 include the tags 1604 receiving wireless signals from the anchors 1602 such that the anchors 1602 are transmitters and the tags 1604 are receivers. In further examples, the anchors 1602 and the tags 1604 are both transmitters and receivers (i.e., transceivers that allow for two-way, bidirectional communications).

In one illustrative example, the care delivery system 100 uses signal communications between the anchors 1602 and a tag 1604a worn by the patient P to monitor the location and movement of the patient P inside their home. The location of the patient P is detected when the tag 1604a is in close proximity to an anchor 1602.

The location of the patient P can indicate a safety risk, such as when a presence of the caregiver C or the family member F is not detected by the anchor 1602. For example, a safety risk can exist when the patient P exits their bed, leaves the bedroom, and/or enters the bathroom without the caregiver C or the family member F being present when required by a fall prevention protocol. As another example, a safety risk can exist when the anchors 1602a, 1602b detect that the patient P leaving their home without permission or authorization (e.g., patient elopement).

When a potential safety risk is detected by the care delivery system 100, an alert is generated. The alert can be sent by the care delivery system 100 to the computing devices 104 of the caregiver C and/or the family member F via the communications network 110.

In another example, the care delivery system 100 uses signal communications between the anchors 1602 and the tag 1604a worn by the patient P to monitor the movement of the patient P inside the patient P's home. The care delivery system 100 uses the monitored movement of the patient P to determine compliance with a mobility plan that requires the patient P to increase their movements over a period of time. When noncompliance is detected, the care delivery system 100 generates an alert that is sent to the computing devices 104 of the patient P, the caregiver C, and/or the family member F to request the patient P to increase their mobility. Additional types of care plans are contemplated such that the care delivery system 100 can generate a variety of alerts for the patient P to change their behavior to comply with a care plan.

In another example, the care delivery system 100 uses signal communications between the anchors 1602 and tags 1604b, 1604c worn by the caregiver C and the family member F to recognize the presence or absence of the caregiver C and/or the family member F in the patient P's home 101. The care delivery system 100 can generate an alert when the presence of the caregiver C or the family member F is required by a hospital-at-home treatment plan, and the signal communications between the anchors 1602 and the tags 1604b, 1604c detect an absence of the caregiver C and/or the family member F in the patient P's home 101. The alert can be sent by the care delivery system 100 to the computing devices 104 of the caregiver C and/or the family member F via the communications network 110 instructing them to visit the patient P's home 101 to confirm the safety and well-being of the patient P.

In another example, the care delivery system 100 receives signal communications between the anchors 1602 and tags 1604d attached to one or more of the medical devices 140 and the medical equipment 1100 that are delivered to the patient P's home 101 in accordance with the hospital-at-home treatment plan. The care delivery system 100 uses the signal communications to track the location and movement of the medical devices 140 and the medical equipment 1100 to aid a caregiver to locate the devices and equipment such as during a medical intervention or to retrieve of the medical devices 140 and the medical equipment 1100 from the patient P's home 101 after completion of the hospital-at-home treatment plan.

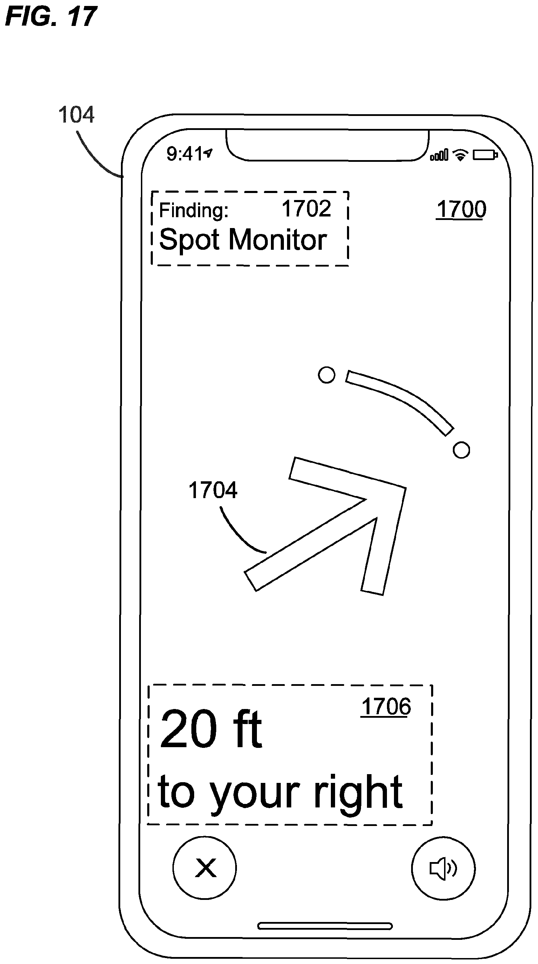

FIG. 17 illustrates an example of an interface 1700 that can be generated by the care delivery system 100 on a computing device 104 to assist location of the medical devices 140 and the medical equipment 1100 in the patient P's home 101. The interface 1700 can display information regarding the presence or absence of the medical devices 140 and/or the medical equipment 1100 inside the patient P's home 101. For example, the interface 1700 can display information 1702 identifying the medical devices 140 or medical equipment 1100 in the patient P's home. The interface 1700 can further include an arrow 1704 and textual information 1706 regarding a direction and/or distance to help guide one or more persons toward the location of the medical devices 140 and the medical equipment 1100 inside the patient P's home 101.

In some examples, when the absence of a medical device or piece of medical equipment is detected, the care delivery system 100 can generate a notification for the caregiver to bring said medical device or piece of medical equipment to the patient P's home prior to their visit. Alternatively, the notification can request to have said medical device or piece of medical equipment delivered to the patient P's home such as by using the drone 1300 or other type of delivery service prior to the caregiver's visit to the patient P's home.

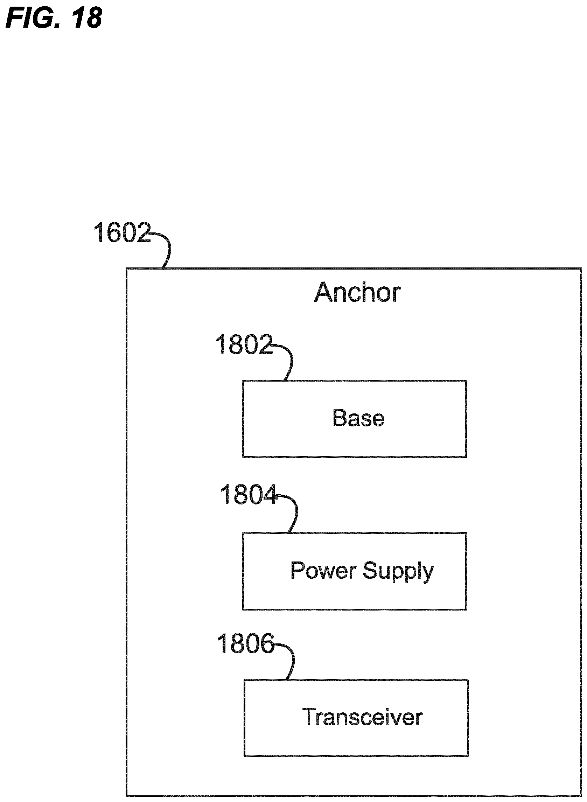

FIG. 18 schematically illustrates an example of an anchor 1602. The anchor 1602 is a fixed reference point that can be temporarily and flexibly deployed inside the patient P's home 101, the container 103, or elsewhere on short notice and for a short period of time such as several days or weeks. For example, the anchor 1602 can be temporarily deployed inside the patient P's home 101 or the container 103 for the duration of a hospital-at-home treatment plan that may last for about 5 to about 10 days. Additionally, the anchor 1602 is removable from the patient P's home 101, the container 103, and elsewhere without causing damage.

In the example shown in FIG. 18, the anchor 1602 includes a base 1802, a power supply 1804, and a transceiver 1806. The base 1802 allows the anchor 1602 to be temporarily and flexibly placed inside the patient P's home 101, the container 103, the clean room 105, and elsewhere without causing damage when removed therefrom. In some example, the base 1802 includes a fastener such as double sided adhesive tape, hook and loop strips, and similar types of fasteners to temporarily attach the anchor 1602 to a wall or other surface without causing damage when the anchor 1602 is removed therefrom. In some examples, the base 1802 includes a pedestal or support structure that allows the anchor 1602 to be supported on a surface such as a piece of furniture or a fixture without being durably attached thereto. In further examples, the base 1802 includes a prongs that allows the anchor 1602 to be plugged into an electrical outlet that can also physically support the anchor 1602 on the wall where the electrical outlet is located.

The power supply 1804 powers the transceiver 1806. In some examples, the power supply 1804 is self-contained such as a battery that is rechargeable or replaceable. In some examples, the power supply 1804 includes a power cord that can be plugged into an electrical outlet inside the patient P's home 101 or the container 103 to receive electrical power.

The transceiver 1806 is powered by the power supply 1804 to detect the presence of a tag 1604. For example, the transceiver 1806 can detect the presence of the tag 1604a worn by the patient P, the tag 1604b worn by the caregiver C, the tag 1604c worn by the family member F, and the tags 1604d attached to the medical devices 140 and medical equipment 1100.

The transceiver 1806 can transmit a signal that a tag 1604 is detected to another computing device for further processing. For example, the transceiver 1806 can transmit a signal that a tag 1604 is detected to the computing device 104 of the patient P, and the computing device 104 can process the signal to determine whether to generate an alert based on a safety risk. In another example, the transceiver 1806 can transmit a signal that a tag 1604 is detected to the computing device 104, and the computing device relays the signal via the communications network 110 to the alert system 114 for further processing. In such example, the alert system 114 determines whether to generate an alert based on a safety risk determined from detection of the tag 1604. In further examples, the transceiver 1806 can directly transmit a signal identifying detection of the tag 1604 to the alert system 114 via the communications network 110.

In some examples, the anchor 1602 includes a sensor that detects when the anchor 1602 is removed or malfunctions before completion of the hospital-at-home treatment plan. In such examples, the transceiver 1806 can send a notification to the alert system 114 for a remote care team to evaluate and resolve the issue of the missing or faulty anchor.

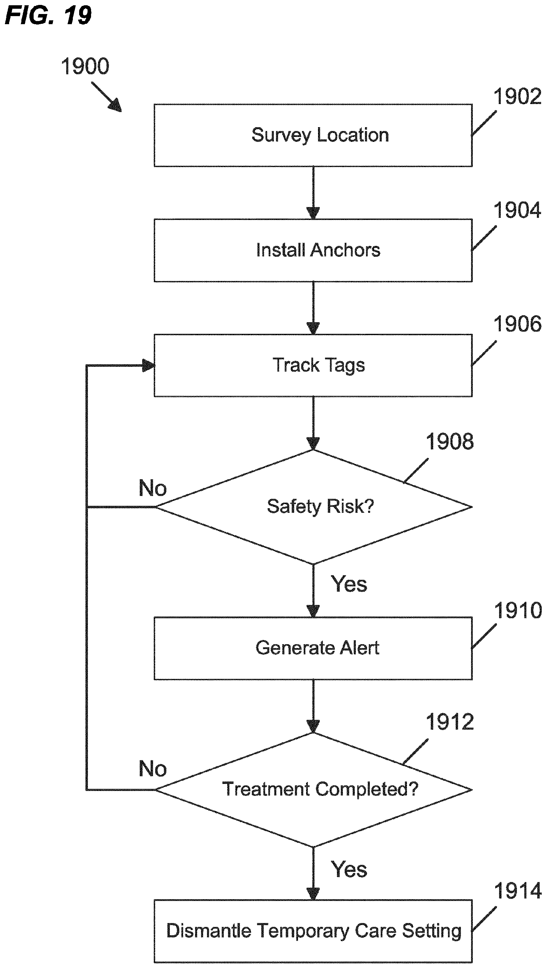

FIG. 19 schematically illustrates an example of a method 1900 of monitoring objects inside a temporary care setting such as the patient P's home 101, a container 103, or a clean room 105 temporarily installed in the patient's home. The method 1900 can be performed by the care delivery system 100 of FIG. 16. In certain examples, the method 1900 is performed to track the location and movement of objects during a hospital-at-home treatment plan.

The method 1900 includes an operation 1902 of surveying the temporary care setting to determine placements for installing one or more of the anchors 1602. In examples where the temporary care setting is the patient P's home 101, the temporary care setting is unique based on the layout of the patient P's home such that it differs from other temporary care settings.

Next, the method 1900 includes an operation 1904 of installing one or more of the anchors 1602 inside the temporary care setting such as the patient P's home 101, the container 103, or the clean room 105. In accordance with the examples described above, the anchors 1602 can be temporarily and flexibly placed inside the patient P's home 101, the container 103, the clean room 105, and elsewhere without causing damage when removed therefrom.

Next, the method 1900 includes an operation 1906 of tracking the tags 1604 attached to one or more objects inside the temporary care setting such as the patient P, the caregiver C, the family member F, and the medical devices 140 and medical equipment 1100. By tracking the tags 1604, the presence, location, and/or movement of the patient P, the caregiver C, the family member F, and the medical devices 140 and medical equipment 1100 can be monitored.

Next, the method 1900 includes an operation 1908 of determining whether a safety risk exists based on the presence or absence of the patient P, the caregiver C, the family member F inside the temporary care setting. For example, a safety risk can exist when the caregiver C and family member are detected as not being present in the temporary care setting such that the patient P is left alone. As another example, a safety risk can exist when the patient P exits their bed, leaves the bedroom, and/or enters the bathroom without the caregiver C or the family member F being present next to them when required by a fall prevention protocol. As another example, a safety risk can exist when the anchors 1602a, 1602b detect that the patient P has left their home without permission or authorization (e.g., patient elopement). In further examples, a safety risk can be based on a physiological event such as one or more elevated vital signs.

When no safety risk is determined to exist (i.e., "No" at operation 1908), the method 1900 returns to the operation 1906 of tracking the tags 1604 attached to one or more objects inside the temporary care setting. When a safety risk is determined to exist (i.e., "Yes" at operation 1908), the method 1900 proceeds to an operation 1910 of generating an alert. In accordance with the examples described above, the alert can be sent to the computing devices 104 of the patient P, the caregiver C, and the family member F by the communications network 110. The alert can instruct the patient P to stop doing an activity that can be harmful to their health such as exiting their bed without assistance from the caregiver C or the family member F. As another example, the alert can instruct the caregiver C or the family member F to go to the temporary care setting when their absence is detected by the anchors 1602.

Next, the method 1900 includes an operation 1912 of determining whether a care plan for the patent P is complete. For example, the care plan can be a hospital-at-home treatment plan that lasts several days such as from about 5 days to about 10 days.

When the care plan is not complete (i.e., "No" at operation 1912), the method 1900 returns to the operation 1906 of tracking the tags 1604 attached to one or more objects inside the temporary care setting. When the care plan is complete (i.e., "Yes" at operation 1912), the method 1900 proceeds to an operation 1914 of dismantling the temporary care setting. Operation 1914 can include generating the interface 1700 to assist removal of the medical devices 140 and the medical equipment 1100 that were delivered to the patient P's home 101. Operation 1914 can further removing the anchors 1602 without causing damage to the patient P's home. In certain examples, operation 1914 can include loading the container 103 onto a truck for removal away from the driveway, front yard, backyard and the like of the patient P's home 101.

Referring now to FIGS. 1 and 16, when a caregiver such as the second caregiver C2 visits a temporary care setting such as the patient P's home 101, a container 103 deployed outside of the patient P's home, or a clean room 105 assembled inside the patient P's home, the caregiver may not know the location of the patient P and/or the location of one or more of the medical devices 140 and medical equipment 1100 that have been delivered to the temporary care setting due to the caregiver C's unfamiliarity of the temporary care setting. For example, when the caregiver visits the patient P's home 101 for the first time, they may be unfamiliar with the layout of the patient P's home 101. This can cause confusion and take time away from providing care to the patient P while the caregiver familiarizes themselves with the temporary care setting.

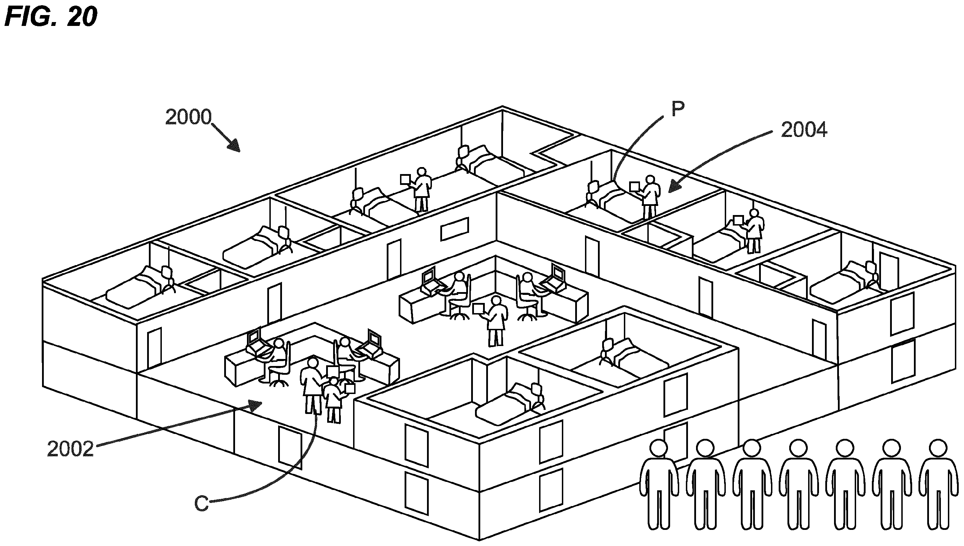

As will be described in more detail below, the care delivery system 100, including the anchors 1602 and the tags 1604 described above, can be installed in an environment such as the patient P's home 101, the container 103, the clean room 105, or a permanent acute care setting 2000 (see FIG. 20) to help the caregiver identify and locate the patient and relevant equipment in the environment prior to the caregiver visiting the environment, and thereafter, provide an easy way for the caregiver to track and identify the location of the patient and the relevant equipment.

FIG. 20 illustrates an example of the permanent acute care setting 2000. As shown in FIG. 20, the caregiver C is located in a work area 2002 such as a nurse's station, while the patient P is located in a room 2004. In the example shown in FIG. 20, the permanent acute care setting 2000 includes multiple rooms spread across the entire floor. In further examples, the permanent acute care setting 2000 can include multiple floors and/or multiple buildings.

In the permanent acute care setting 2000, it is desirable to familiarize the caregiver C with the location of the patient P and of the medical devices needed for providing care to the patient P, especially when the permanent acute care setting 2000 is a large floor or wing of a hospital. Familiarizing the caregiver C with the location of the patient P and of the medical devices can reduce the steps needed to be taken by the caregiver C to initiate a clinical workflow, and can thereby increase the caregiver C's productivity and improve patient satisfaction.

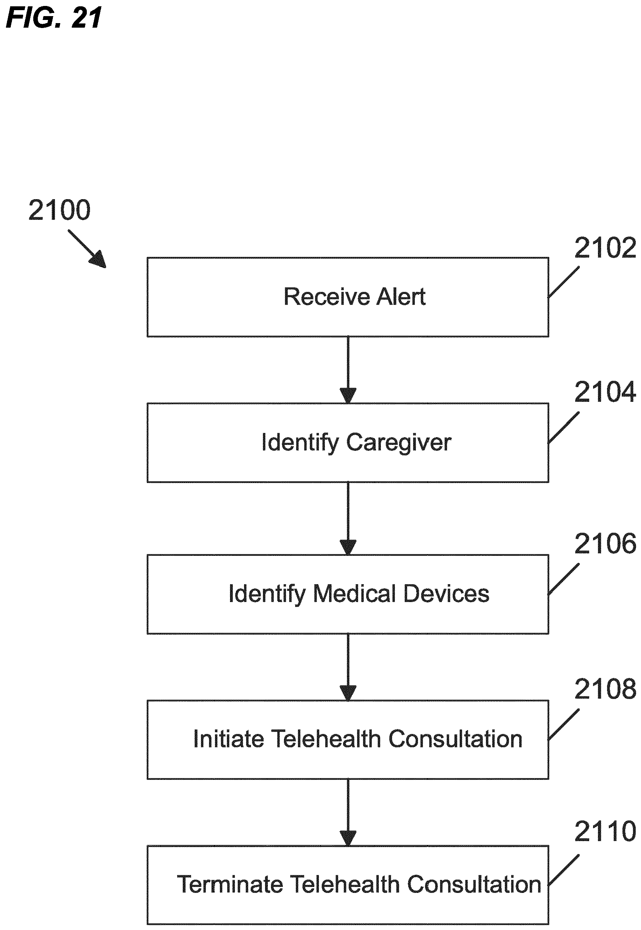

FIG. 21 schematically illustrates an example of a method 2100 of providing location based automation for initiating a clinical workflow. The method 2100 when performed can reduce the steps needed to initiate a clinical workflow in both a temporary care setting (e.g., the patient P's home 101, a container 103 deployed outside of the patient P's home, or a clean room 105 assembled inside the patient P's home) and in the permanent acute care setting 2000.

The method 2100 includes an operation 2102 of receiving an alert of a safety risk. In accordance with the examples described above, the safety risk can be based on the location of the patient P detected by the anchors 1602 when a presence of another caregiver or family member is not detected by the anchors 1602. For example, a safety risk can exist when the anchors 1602 detect that the patient P has exited their bed, left their bedroom, and/or entered the bathroom without another caregiver or family member being present when required by a fall prevention protocol. As another example, a safety risk can exist when the anchors 1602a, 1602b detect that the patient P has left their home without permission or authorization (e.g., patient elopement). In accordance with further examples, the safety risk can be physiological variables that fall outside of an upper or lower limit set for the patient P, detection by one or more sensors on the bed where the patient P is resting that detect a bed exit, or a nurse call placed by the patient P.

Next, the method 2100 includes an operation 2104 of identifying a caregiver to route to the patient P based on the location of the caregiver C relative to the patient P. For example, operation 2104 can identify the caregiver that is in closest proximity to the patient P. In examples where the method 2100 is performed in a temporary care setting such as in the patient P's home 101, operation 2104 can identify a caregiver who is located in the same neighborhood as the patient P's home 101. In examples where the method 2100 is performed in a permanent care setting, operation 2104 can identify a caregiver who is in the same hallway as the patient P's room. In some examples, operation 2104 identifies a caregiver based on their location relative to the patient P and also to one or more medical devices and equipment necessary for the treatment of the patient P. In some examples, the location of the caregiver relative to the patient P and to one or more medical devices and equipment is determined by detection of a tag worn by the caregiver by the anchors 1602 positioned in the temporary or permanent care settings. In other examples, the location of the caregiver relative to the patient P can be determined by a satellite-based radionavigation system such as the Global Positioning System (GPS).

Next, the method 2100 includes an operation 2106 of identifying the location of the one or more medical devices and equipment necessary for the treatment of the patient P. In some examples, the location of the one or more medical devices and equipment is included in the alert 300 routed to the caregiver while they are on their way to the patient P. In further examples, operation 2106 can include generating on the computing device 104 of the caregiver the interface 1700 shown in FIG. 17 to guide the caregiver to the medical devices and equipment while on their way to visit the patient P. In some further examples, operation 2106 can include actuating a speaker of a medical device to provide an audible tone that the caregiver can hear to make it easier for the caregiver to locate the device. This can reduce the time needed for the caregiver to reach the patient P with the correct medical devices and equipment, thereby increasing the caregiver's productivity. In some examples, the location of the medical devices and equipment relative to the caregiver is determined by detection of tags attached to the medical devices and equipment by the anchors 1602 positioned in the temporary or permanent care settings.

In some examples, when the absence of a medical device or piece of medical equipment is detected, the care delivery system 100 can generate a notification for the caregiver to bring said medical device or piece of medical equipment to the patient P's location prior to travelling to the location. Alternatively, the notification can request to have said medical device or piece of medical equipment delivered to the patient P's location prior to the caregiver's visit.

Next, the method 2100 includes an operation 2108 of automatically initiating a telehealth consultation while the caregiver is being routed to the patient P. In some examples, the telehealth consultation is initiated between the caregiver who is being routed to the patient P (e.g., the second caregiver C2 in FIG. 1). In alternative examples, the telehealth consultation is initiated between a remote caregiver and the patient P (e.g., the first caregiver C1 in FIG. 1).

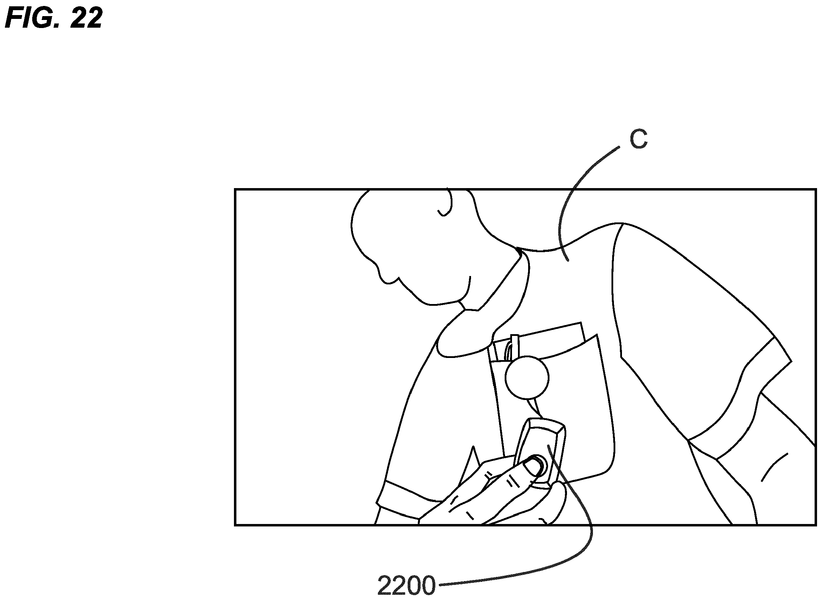

FIG. 22 illustrates an example of a badge 2200 worn by a caregiver C who is being routed to the patient P. Operation 2108 can include automatically operating the badge 2200 to initiate two-way audio communication between the caregiver C and the patient P. In some examples, the badge 2200 or a computing device 104 carried by the caregiver C can be automatically operated provide video communications between the caregiver C and the patient P.

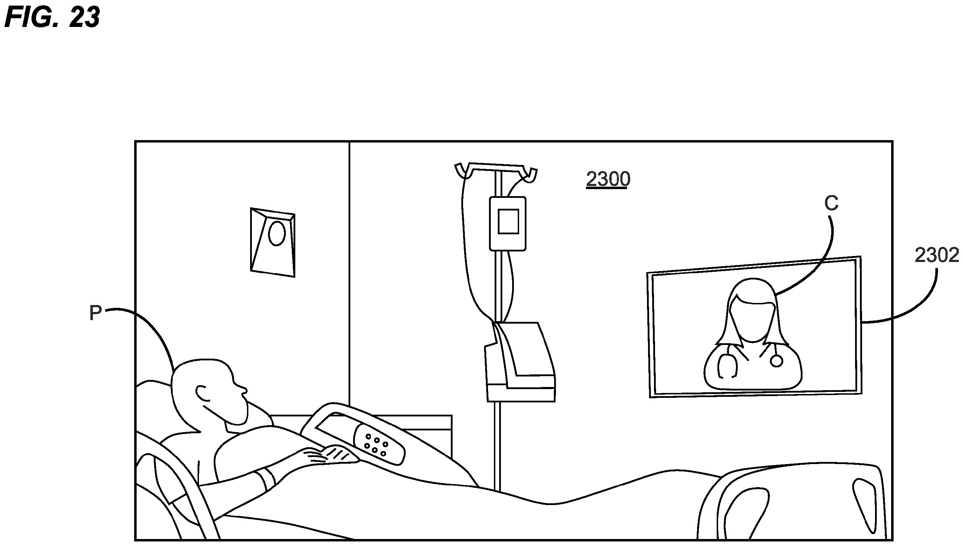

FIG. 23 illustrates an example of a video telehealth consultation on a display device 2302 located in a room 2300 that is initiated between the patient P and a caregiver C. The video telehealth consultation is initiated while the caregiver C is being routed to the location of the patient P. In some examples, the telehealth consultation includes the caregiver who is being routed to the patient P (e.g., the second caregiver C2 in FIG. 1). In alternative examples, the telehealth consultation includes a remote caregiver (e.g., the first caregiver C1 in FIG. 1).

Referring back to FIG. 21, the method 2100 includes an operation 2110 of automatically terminating or transferring the telehealth consultation when it is detected that the caregiver is in close proximity to the patient P. For example, when the caregiver C enters the bedroom where the patient P is located inside the patient P's home 101 (see FIG. 16), operation 2110 automatically terminates the telehealth consultation because it is no longer needed. Similarly, when the caregiver C arrives to the room 2004 of the patient P inside the permanent acute care setting 2000 (see FIG. 20), the telehealth consultation is automatically terminated.

In some examples, instead of terminating the telehealth consultation, operation 2110 includes transferring the telehealth consultations from the caregiver C to another type of caregiver such as a medical specialist who is remotely located with respect to the patient P and the caregiver C. In such examples, the medical specialist can instruct the caregiver C to perform a clinical workflow while the caregiver C is present in the same location of the patient P.

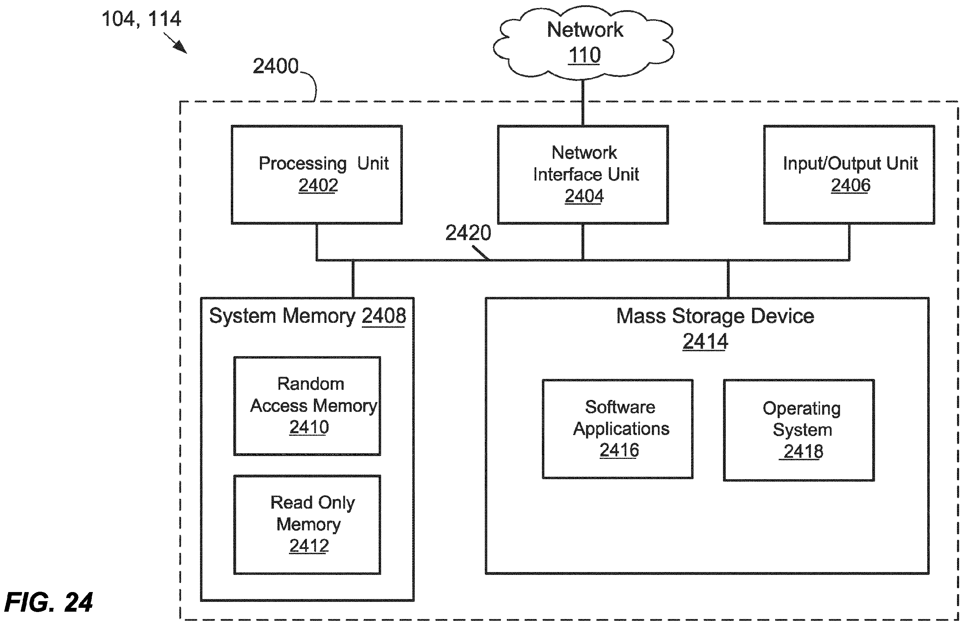

FIG. 24 schematically illustrates an example computing device 2400 which can be used to implement aspects of the care delivery system 100, including the functions of the computing devices 104 and alert system 114 described above. The computing device 2400 includes a processing unit 2402, a system memory 2408, and a system bus 2420 that couples the system memory 2408 to the processing unit 2402. The processing unit 2402 is an example of a processing device such as a central processing unit (CPU). The system memory 2408 includes a random-access memory ("RAM") 2410 and a read-only memory ("ROM") 2412. A basic input/output logic containing the basic routines that help to transfer information between elements within the computing device 2400, such as during startup, is stored in the ROM 2412.

The computing device 2400 can also include a mass storage device 2414 that is able to store software instructions and data. The mass storage device 2414 is connected to the processing unit 2402 through a mass storage controller (not shown) connected to the system bus 2420. The mass storage device 2414 and its associated computer-readable data storage media provide non-volatile, non-transitory storage for the computing device 2400.

Although the description of computer-readable data storage media contained herein refers to a mass storage device, it should be appreciated by those skilled in the art that computer-readable data storage media can be any available non-transitory, physical device or article of manufacture from which the device can read data and/or instructions. The mass storage device 2414 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, or any other medium which can be used to store information, and which can be accessed by the device.

The computing device 2400 may operate in a networked environment using logical connections to remote network devices, including the alert system 114, through the communications network 110, such as a local network, the Internet, or another type of network. The computing device 2400 connects to the communications network 110 through a network interface unit 2404 connected to the system bus 2420. The network interface unit 2404 may also be utilized to connect to other types of networks and remote computing systems.

The computing device 2400 can also include an input/output controller 2406 for receiving and processing input from a number of input devices. Similarly, the input/output controller 2406 may provide output to a number of output devices.

The mass storage device 2414 and the RAM 2410 can store software instructions and data. The software instructions can include an operating system 2418 suitable for controlling the operation of the device. The mass storage device 2414 and/or the RAM 2410 also store software instructions 2416, that when executed by the processing unit 2402, cause the device to provide the functionality of the device discussed in this document.

## Claims

1. A care delivery system comprising:
one or more sensors monitoring a patient in a first location;
at least one processing device in communication with the one or more sensors; and
at least one computer readable data storage device storing software instructions that, when executed by the at least one processing device, cause the care delivery system to:
detect an event from the one or more sensors, the event indicating a safety risk to the patient in the first location;
determine a clinical workflow based on the safety risk and one or more rules defined by a healthcare facility in a second location remote from the first location;
initiate a telehealth consultation based on the clinical workflow, the telehealth consultation including the patient and a first caregiver in the second location;
generate an alert identifying the safety risk; and
route the alert to a second caregiver based on the clinical workflow.

2. The system of claim 1, wherein the software instructions, when executed by the at least one processing device, further cause the care delivery system to:
escalate the alert to have a higher priority level based on the telehealth consultation; and
update the clinical workflow based on the higher priority level.

3. The system of claim 2, wherein the clinical workflow is updated to route the alert to a third caregiver, wherein the first caregiver is an inpatient medical professional stationed in the healthcare facility, wherein the second caregiver is a family member of the patient, and wherein the third caregiver is an outpatient medical professional.

4. The system of any preceding claim, wherein the one or more sensors include a wearable device that records physiological data and environmental events of the patient.

5. The system of claim 4, wherein the one or more sensors further include a pad positioned on a floor next to where the patient is resting on a bed.

6. The system of either claim 4 or claim 5, wherein the one or more sensors further include camera worn by the patient, and wherein the camera records a video stream.

7. The system of claim 6, wherein the software instructions, when executed by the at least one processing device, further cause the care delivery system to:
retrieve the video stream from the camera;
segment a window from the video stream, the window including portions of the video stream recorded before and after the event is detected; and
generate the alert to include the window from the video stream and the physiological data recorded by the one or more sensors during the window.

8. The system of any preceding claim, wherein the first location is the patient's home, or a clean room temporarily assembled inside the patient's home.

9. The system of any preceding claim, wherein the first location is a container temporarily deployed outside of the patient's home, the container including:
a power source;
a water supply;
a communications system to facilitate the telehealth consultation;
a ventilation system to filter air and create negative air pressure inside the container; and
a sterilization unit to sterilize the container for re-deployment and use by another patient.

10. The system of any preceding claim, wherein the one or more sensors include anchors positioned inside the first location, and tags worn by the patient and the second caregiver.

11. The system of claim 10, wherein the event indicates the safety risk when the patient exits a bed, leaves a room, or enters another room without the second caregiver being present, and when presence of the second caregiver is required by a fall prevention protocol.

12. The system of any preceding claim, wherein the software instructions, when executed by the at least one processing device, further cause the care delivery system to:
generate an interface on a computing device displaying information that identifies a location of a tag attached to a medical device inside the first location, and an arrow pointing to the location of the medical device inside the first location.

13. The system of any preceding claim, wherein the software instructions, when executed by the at least one processing device, further cause the care delivery system to:
identify a location of a medical device for treating the patient; and
include the location of the medical device in the alert routed to the second caregiver.

14. The system of any preceding claim, wherein the software instructions, when executed by the at least one processing device, further cause the care delivery system to:
terminate the telehealth consultation when the second caregiver enters the first location.

15. The system of any preceding claim, wherein the software instructions, when executed by the at least one processing device, further cause the care delivery system to:
transfer the telehealth consultation to a specialist who is remotely located with respect to the patient and the second caregiver when the second caregiver enters the first location.
